# EUROPEAN PATENT APPLICATION

(11) **EP 2 725 344 A1**
(43) Date of publication of application: **30.04.2014**
(21) Application number: 12815361.6
(22) Date of filing: 03.07.2012
(51) Int. Cl.: G01N 21/27

(54) **TARGET SUBSTANCE DETECTION CHIP, TARGET SUBSTANCE DETECTION PLATE, TARGET SUBSTANCE DETECTION DEVICE AND TARGET SUBSTANCE DETECTION METHOD**

(30) Priority: 15.07.2011 JP 2011157242; 15.07.2011 JP 2011157243
(71) Applicant: National Institute of Advanced Industrial Science and Technology, Tokyo 100-8921 (JP)
(72) Inventor: FUJIMAKI Makoto, Tsukuba-shi, Ibaraki 305-8565 (JP); FUKUDA Nobuko, Tsukuba-shi, Ibaraki 305-8565 (JP); NOMURA Kenichi, Tsukuba-shi, Ibaraki 305-8565 (JP); NAGAI Hidenori, Ikeda-shi, Osaka 563-8577 (JP); OOIE Toshihiko, Takamatsu-shi, Kagawa 761-0395 (JP)
(74) Representative: Hill, Christopher Michael
(86) International application number: PCT/JP2012/066964
(87) International publication number: WO 2013/011831

(57) **Abstract**

[Problem] To provide a target substance detection chip, a target substance detection device, and a target substance detection method, that can be manufactured easily in a small size at low costs with reduction of the number of parts involved in the detection chip constituted by an optical prism and a detection plate used for a SPR sensor and an optical waveguide mode sensor, that can detect a target substance quickly with high sensitivity, and in which an analyte liquid is easily delivered.

[Solution] A target substance detection chip of the present invention includes: a plate-like transparent base portion which allows light to pass therethrough; and a flow path which is formed in one surface of the transparent base portion as a groove and through which an analyte liquid verifying a presence of a target substance is delivered in a length direction of the groove, wherein the flow path is formed such that at least an electric field enhancement layer is disposed on an inner surface of a groove portion formed to at least partly have inclined surfaces appearing in cross section to be inclined at a gradient to the surface of the transparent base portion, and wherein a part or entirety of an uppermost surface of the groove which contacts the analyte liquid serves as a detection surface for the target substance.

## Description

### Technical Field

The present invention relates to a target substance detection chip configured to detect a target substance contained in an analyte liquid using an optical waveguide mode and a surface plasmon resonance, a target substance detection plate including the target substance detection chip, a target substance detection device, and a target substance detection method using the target substance detection device.

### Background Art

Recently, portable, easy-to-handle detection devices capable of highly sensitively detecting a target substance are required in various fields such as health checkup, drug development, early detection of diseases and contagions, detection of environmental pollutions, antiterror measures, etc.

SPR sensors utilizing a surface plasmon resonance (SPR) and optical waveguide mode sensors utilizing an optical waveguide mode have been known as sensors that are small enough in size to be portable and are capable of measuring various substances contained in a liquid (see NPLs 1 to 19 and PTLs 1 to 7). These sensors have been used for detection of various biomarkers attributable to diseases or detection of viruses, selective detection of biomaterials such as proteins, evaluation of environmental pollutions due to heavy metals or oils present in the environment, and detection of poisonous substances, illegal drugs, or explosives used in terrorism.

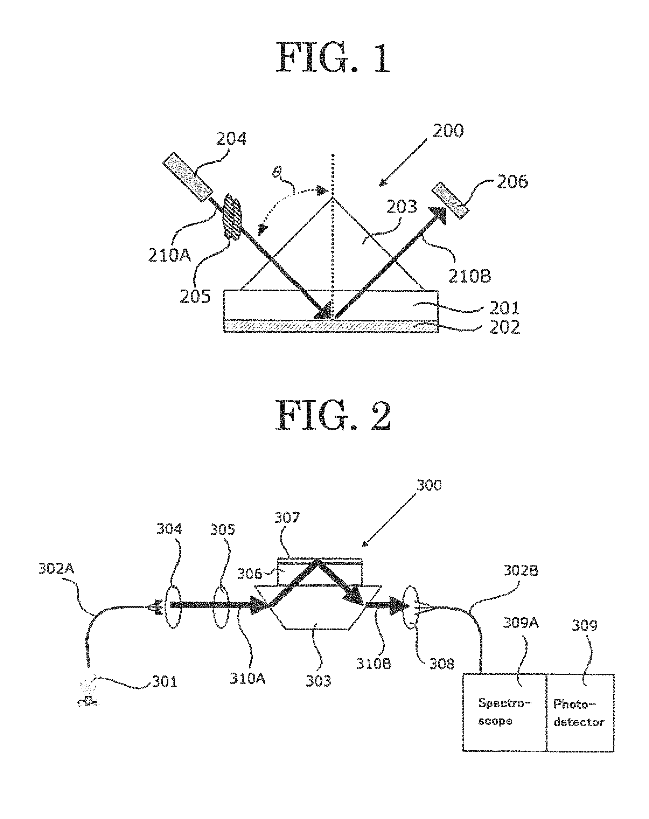

Fig. 1 illustrates an exemplary configuration of the most popular SPR sensor 200 in Kretschmann configuration. The SPR sensor 200 has a configuration including the thin metal layer 202 which is formed by vapor-depositing metals such as gold, silver, and aluminum on the transparent substrate 201 and the optical prism 203 which is adhered to a surface of the transparent substrate 201 opposite to a surface on which the thin metal layer 202 is formed; and has a function of polarizing laser light irradiated from the light source 204 by the polarizing plate 205 and irradiating the polarized light to the transparent substrate 201 through the optical prism 203. The incident light 210A is made incident under a condition at which total reflection occurs. A surface plasmon resonance appears at a certain incident angle by an evanescent wave formed when the incident light 210A is transmitted to a metal surface-side. The incident angle θ is appropriately changed with actuation of the optical system. When the surface plasmon resonance appears, the evanescent wave is absorbed by surface plasmon, therefore, reflected light near the incident angle is significantly decreased in intensity. A condition under which the surface plasmon resonance appears varies depending on the dielectric constant in the proximity of the surface of the thin metal layer 202. Therefore, when a substance adsorbs to, approaches, desorbs from, or changes in property on the surface of the thin metal layer 202, the intensity of the reflected light 210B changes. Thus, when a sample to be detected binds to or adsorbs on the surface of the thin metal layer 202 to thereby change the dielectric constant, the reflection property of the incident light 210A also changes. Accordingly, the sample to be detected can be detected by monitoring, using the optical detector 206, a change in intensity of the reflected light 210B reflected from the thin metal layer 202.

A spectral measurement method has been reported in which an optical system in a SPR sensor is simplified and small-sized (see NPLs 6 and 7). Fig. 2 illustrates a schematic view of the SPR sensor 300 provided with the optical system according to the report in NPL 6. The incident light 310A is directed from the light source 301 to in front of the optical prism 303 via the optical fiber 302A, made into collimated light by the collimator lens 304, and then p-polarized by the polarizing plate 305, followed by being incident on the optical prism 303. This incident light 310A is irradiated to the thin metal layer 307 on the transparent substrate 306, the glass substrate being arranged so as to adhere to the optical prism 303; and directed through the condensing lens 308 to the photodetector 309 via the optical fiber 302B, as the reflected light 310B which is reflected from the thin metal layer 307. Here, the photodetector 309 is provided with the spectroscope 309A, and has a function of measuring the reflection spectrum of the reflected light 310B. The SPR sensor 300 is similar to the SPR sensor 200 in that a change in the dielectric constant can be detected by measuring the reflection spectrum caused by the change in the dielectric constant in the proximity of a surface of the thin metal layer 307. However, it is different from the SPR sensor 200 in that the reflected light 310B is wavelength-resolved, and then measured for the spectrum thereof without changing the incident angle of the incident light 310A to the thin metal layer by actuating the optical system, which allows the optical system to be simplified and the device to be small-sized.

An optical waveguide mode sensor is a sensor which is similar to the SPR sensor in configuration and which also detects adsorption of a substance or change in the dielectric constant at a detecting surface of the sensor. The optical waveguide mode sensor has been known to be capable of using an optical system equivalent to any optical systems that can be used in the SPR sensors.

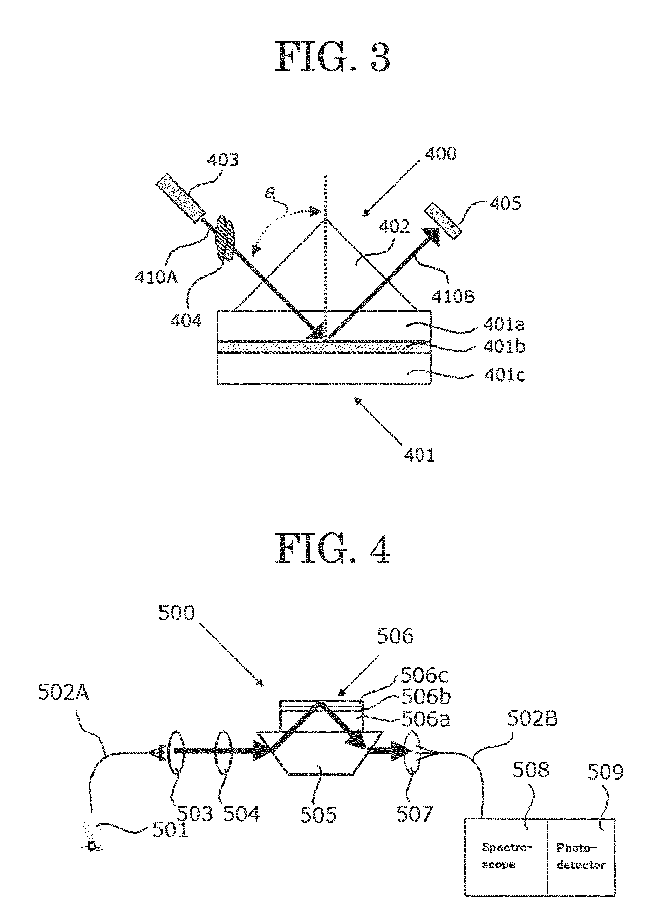

Fig. 3 illustrates the optical waveguide mode sensor 400 having a similar configuration to the Kretschmann configuration. The optical waveguide mode sensor 400 uses the detection plate 401 consisting of the transparent substrate 401a, the thin layer 401b composed of a metal layer or a semiconductor layer coated on the transparent substrate, and the optical waveguide layer 401c formed on the thin layer 401b. Further, the optical prism 402 is adhered, via a refractive index-matching oil, to the surface of the detection plate 401 opposite to the surface on which the optical waveguide layer 401c is formed. Incident light 410A is irradiated from the light source 403, polarized by the polarizing plate 404, and then irradiated to the detection plate 401 through the optical prism 402. The incident light 410A is incident on the detection plate 401 under a condition at which total reflection occurs. Upon coupling of the incident light 410A with the optical waveguide mode (may be referred to as leakage mode or leaky mode) at a certain incident angle θ, the optical waveguide mode is excited to thereby significantly change the reflected light in intensity near the incident angle. Such a condition for exciting optical waveguide mode varies depending on the dielectric constant in the proximity of the surface of the optical waveguide layer 401c. Therefore, the reflected light 410B changes in intensity when a substance is adsorbed onto, approaches, desorbs from, or changes in property on a surface of the optical waveguide layer 401c. These phenomena such as adsorption, approaching, desorption, or change in property on the surface of the optical waveguide layer 401c can be detected by measuring the change in intensity with the optical detector 405.

Fig. 4 illustrates a schematic view of an optical waveguide mode sensor 500, which is an optical waveguide mode sensor employing the optical system of the SPR sensor 300 illustrated in Fig. 2. A light irradiation means illustrated in Fig. 4 includes a light source 501, an optical fiber 502A, a collimator lens 503, and a polarizing plate 504. Light from the light source 501 enters the optical fiber 502A to be guided to a location from which it can be easily let into an optical prism 505. The light emitted from the optical fiber 502A is set to become collimated light by the collimator lens 503 located at the exit of the optical fiber 502A. This emitted light enters the optical prism 505 after it is polarized to a desired polarization state by the polarizing plate 504. The light entered the optical prism 505 is reflected by a detection plate 506 and emitted from the optical prism 505 as reflected light, and after this, condensed by the condensing lens 507 to be collected into an optical fiber 502B, so that the reflection intensity or the reflection spectrum thereof can be measured by a spectroscope 508 and an optical detector 509. The detection plate 506 has a configuration in which a thin layer 506b made of a metal layer or a semiconductor layer and an optical waveguide layer 506c are provided in this order on a transparent substrate 506a. The optical prism 505 is optically attached to a surface of the detection plate 506 opposite to the surface thereof where the optical waveguide layer 506c is provided. In a measurement of a property, e.g., a reflected light spectrum to be observed after incident light is reflected by the detection plate 506, a phenomenon occurs that light included in the incident light and present within a specific wavelength band satisfies a condition under which an optical waveguide mode, which is to propagate locally inside and in the vicinity of the optical waveguide layer 506c formed on the surface of the detection plate 506, is excited to thereby significantly change the intensity of reflection of this wavelength band. Since this optical waveguide mode excitation condition varies depending on the dielectric constant in the proximity of the surface of the optical waveguide layer 506c of the detection plate 506, a change in the dielectric constant in the proximity of the surface of the optical waveguide layer 506c causes a change in the reflection spectrum. Therefore, by measuring changes in the reflection spectrum or changes in the intensity of the reflected light present within the specific wavelength band, it is possible to detect, with the optical detector 509, the cause of the changes in the dielectric constant in the proximity of the surface of the optical waveguide layer 506c, e.g., adsorption, approaching, desorption, changes in property of a substance.

Further, it has been reported that an optical waveguide mode sensor can tremendously improve its detection sensitivity, if the surface area of its detection surface is increased with formation of nano-pores in the optical waveguide layer (see, e.g., PTLs 4 and 5, and NPLs 10 to 13).

SPR sensors and optical waveguide mode sensors also have an effect of enhancing luminescence of a substance capable of optical excitation luminescence, e.g., a fluorochrome (hereinafter referred to as fluorescent substance), when the fluorescent substance is brought into contact with or neared to the detection surface. This effect is often utilized for signal amplification for detection of a substance. For example, when a desired specific substance is captured in the proximity of the surface of the thin metal layer 202 of Fig. 1, the method of measuring changes in the property of the reflected light illustrated in Fig. 1 may not obtain a sufficient signal, if this specific substance is a very small substance, exists in a very small amount, or has a dielectric constant that is almost the same as the surrounding medium. For such a case, a fluorescent substance may be attached to the specific substance captured, and used as a label. The attached fluorescent substance will emit light with an intensity increased by an electric field enhancing effect of a plasmon excited by excitation light. Therefore, the capture of the specific substance can be indirectly detected at high sensitivity. This effect can likewise be obtained in the proximity of the surface of the thin metal layer 307 of Fig. 2, in the proximity of the surface of the optical waveguide layer 401c of Fig. 3, and in the proximity of the surface of the optical waveguide layer 506c of Fig. 4.

Here, in any of the cases illustrated in Fig. 1 to Fig. 4, luminescence from the fluorescent substance mainly takes place to a side of the detection plate opposite to the side irradiated with the excitation light, i.e., to the side on which the fluorescent substance is attached. Therefore, in order to detect this luminescence, a device for detecting the luminescence, e.g., a photodetector such as a CCD, a photomultiplier tube, and a photodiode is placed at the detection surface side of the detection plate, i.e., at the side opposite to the surface on which the prism is provided.

As the SPR sensors and optical waveguide mode sensors, various types of products have already been on sale and widely used. For general measurements, in addition to such a prism and detection plate as illustrated in Fig. 1 to Fig. 4, a delivery path for delivering a detection target substance to the surface of the detection surface needs to be provided to the surface of the detection plate. For example, when the analyte is a liquid, a flow path needs to be provided. This will increase the number of parts involved, and bring about a problem that handling is not easy.

Further, in actual use, the prism, the detection plate, and the delivery path need to be used by being joined together. When the detection plate and the delivery path are replaced for every detection, this joining step needs to be done every time and brings about a problem that the system will be complicated.

Furthermore, in terms of a part, the prism has a problem that it generally requires high-precision polishing and is expensive.

A biochip disclosed in PTL 7 can be raised as an integrally formed example of a prism, a detection plate, and a delivery path. This biochip includes a substrate in which a fine fluid channel is formed as a delivery path, and includes a plurality of wedge-shaped sharpened tip portions formed from first and second inclined surfaces in the fine fluid channel. On the inclined surfaces of the sharpened tip portions, there are formed a metal layer in which a surface plasmon may be excited, and a dielectric layer on which a capture molecule is secured that forms specific binding with the target molecule labeled with a fluorescent material. When the target substance is secured on the dielectric layer, a fluorescence is detected from the fluorescent material that is excited through a surface plasmon.

According to this biochip, the number of parts involved can be reduced, because respective parts that have the functions of a prism, a detection plate, and a delivery path are formed integrally with the substrate.

However, in this biochip, the inclined surfaces of the sharpened tip portions are formed to face the direction from which the analyte liquid is delivered through the fine fluid channel. Therefore, the sharpened tip portions block the delivery of the analyte liquid, and bring about a problem that the analyte liquid is difficult to deliver throughout the fine fluid channel.

Further, in this biochip, the sharpened tip portions, which constitute the detection surface for the target molecule, and the fine fluid channel serving as the delivery path are formed independently. Therefore, there is still a problem that the manufacture cost of the system is high because the system is complicated.

Furthermore, with the sharpened tip configuration of the detection surface, reflected light incident to the first inclined surface of the sharpened tip portions is reflected on the facing second inclined surface. Therefore, presence of the target molecule on the first inclined surface cannot be detected with the use of the optical systems of Fig. 1 to Fig. 4, which are configured to detect based on changes in a property of reflected light.

What is more, establishment of an efficient detection method is required, by providing such a prism, detection plate, and delivery path on a plate.

### Citation List

### Patent Literature

PTL 1: Japanese Patent (JP-B) No. 4581135
PTL 2: JP-B No. 4595072
PTL 3: Japanese Patent Application Laid-Open (JP-A) No. 2007-271596
PTL 4: JP-A No. 2008-46093
PTL 5: JP-A No. 2009-85714
PTL 6: International Publication No. 2010/87088
PTL 7: JP-A No. 2010-145408

### Non-Patent Literature

NPL 1: W. Knoll, MRS Bulletin 16, pp. 29-39 (1991)
NPL 2: W. Knoll, Annu. Rev. Phys. Chem. 49, pp. 569-638 (1998)
NPL 3: H. Kano and S. Kawata, Appl. Opt. 33, pp. 5166-5170 (1994)
NPL 4: C. Nylander, B. Liedberg, and T. Lind, Sensor. Actuat. 3, pp. 79-88 (1982/83)
NPL 5: K. Kambhampati, T. A. M. Jakob, J. W. Robertson, M. Cai, J. E. Pemberton, and W. Knoll, Langmuir 17, pp. 1169-1175 (2001)
NPL 6: O. R. Bolduc, L. S. Live, and J. F. Masson, Talanta 77, pp. 1680-1687 (2009)
NPL 7: I. Stammler, A. Brecht, and G. Gauglitz, Sensor. Actuat. B54, pp 98-105 (1999)
NPL 8: M. Osterfeld, H. Franke, and C. Feger, Appl. Phys. Lett. 62, pp. 2310-2312 (1993)
NPL 9: E. F. Aust and W. Knoll, J. Appl. Phys. 73, p. 2705 (1993)
NPL 10: M. Fujimaki, C. Rockstuhl, X. Wang, K. Awazu, J. Tominaga, T. Ikeda, Y. Ohki, and T. Komatsubara, Microelectronic Engineering 84, pp. 1685-1689 (2007)
NPL 11: K. Awazu, C. Rockstuhl, M. Fujimaki, N. Fukuda, J. Tominaga, T. Komatsubara, T. Ikeda, and Y. Ohki, Optics Express 15, pp. 2592-2597 (2007)
NPL 12: K. H. A. Lau, L. S. Tan, K. Tamada, M. S. Sander, and W. Knoll, J. Phys. Chem. B108, pp. 10812 (2004)
NPL 13: M. Fujimaki, C. Rockstuhl, X. Wang, K. Awazu, J. Tominaga, Y. Koganezawa, Y. Ohki, and T. Komatsubara, Optics Express 16, pp. 6408-6416 (2008)
NPL 14: M. Fujimaki, C. Rockstuhl, X. Wang, K. Awazu, J. Tominaga, N. Fukuda, Y. Koganezawa, and Y. Ohki, Nanotechnology 19, pp. 095503-1 - 095503-7 (2008)
NPL 15: M. Fujimaki, C. Rockstuhl, X. Wang, K. Awazu, J. Tominaga, T. Ikeda, Y. Koganezawa, and Y. Ohki, J. Microscopy 229, pp. 320-326 (2008)
NPL 16: M. Fujimaki, K. Nomura, K. Sato, T. Kato, S. C. B. Gopinath, X. Wang, K. Awazu, and Y. Ohki, Optics Express 18, pp. 15732-15740 (2010)
NPL 17: R. P. Podgorsek, H. Franke, J. Woods, and S. Morrill, Sensor. Actuat. B51 pp. 146-151 (1998)
NPL 18: J. J. Saarinen, S. M. Weiss, P. M. Fauchet, and J. E. Sipe, Opt. Express 13, pp. 3754-3764 (2005)
NPL 19: G. Rong, A. Najmaie, J. E. Sipe, and S. M. Weiss, Biosens. Bioelectron. 23, pp. 1572-1576 (2008)

### Summary of Invention

### Technical Problem

The present invention aims to solve the conventional problems described above and to accomplish the following object. That is, an object of the present invention is to provide a target substance detection chip, a target substance detection device, and a target substance detection method, that can be manufactured easily in a small size at low costs with reduction of the number of parts involved in the detection chip constituted by an optical prism and a detection plate used for a SPR sensor and an optical waveguide mode sensor, that can detect a target substance quickly with high sensitivity, and in which an analyte liquid is easily delivered.

Another object of the present invention is to provide a target substance detection plate, a target substance detection device, and a target substance detection method, that can be manufactured easily in a small size at low costs with reduction of the number of parts involved in a detection chip constituted by an optical prism and a detection plate used for a SPR sensor and an optical waveguide mode sensor, that can detect a target substance quickly with high sensitivity, that include a detection chip to which an analyte liquid is easily introduced, and that can measure a target substance efficiently.

### Solution to Problem

Means for solving the above problems are as follows.
<1> A target substance detection chip, including:
   a plate-like transparent base portion which allows light to pass therethrough; and
   a flow path which is formed in one surface of the transparent base portion as a groove and through which an analyte liquid verifying a presence of a target substance is delivered in a length direction of the groove,
   wherein the flow path is formed such that at least an electric field enhancement layer is disposed on an inner surface of a groove portion formed to at least partly have inclined surfaces appearing in cross section to be inclined at a gradient to the surface of the transparent base portion, and
   wherein a part or entirety of an uppermost surface of the groove which contacts the analyte liquid serves as a detection surface for the target substance.
<2> The target substance detection chip according to <1>, wherein a surface of the transparent base portion opposite to the surface of the transparent base portion in which the flow path is formed is formed to be flat.
<3> The target substance detection chip according to <1> or <2>, wherein a right groove side surface and a left groove side surface forming the groove portion are formed to be laterally symmetric.
<4> The target substance detection chip according to any one of <1> to <3>, wherein the electric field enhancement layer is formed such that a surface plasmon excitation layer that causes surface plasmon resonance is disposed on the groove portion.
<5> The target substance detection chip according to <4>, wherein a formation material for the surface plasmon excitation layer contains at least one of gold, silver, copper, platinum, and aluminum.
<6> The target substance detection chip according to <4> or <5>, wherein a surface of the surface plasmon excitation layer is covered with a transparent dielectric.
<7> The target substance detection chip according to any one of <1> to <3>, wherein the electric field enhancement layer is formed of: a thin layer formed of a metal material or a semiconductor material; and an optical waveguide layer formed of a transparent material, the thin layer and the optical waveguide layer being disposed on the groove portion in this order.
<8> The target substance detection chip according to <7>, wherein the metal material contains at least one of gold, silver, copper, platinum, and aluminum.
<9> The target substance detection chip according to <7>, wherein the semiconductor material is silicon.
<10> The target substance detection chip according to any one of <7> to <9>, wherein the optical waveguide layer is formed of silica glass.
<11> The target substance detection chip according to any one of <1> to <10>, wherein the detection surface is surface-treated so as to capture the target substance.
<12> The target substance detection chip according to any one of <1> to <11>, wherein a lid is disposed on the surface of the transparent base portion in which the flow path is formed so as to block an opening of the flow path.
<13> The target substance detection chip according to <12>, wherein the lid includes one of a seal material and a plate material formed of one of a transparent resin material and a transparent glass material.
<14> The target substance detection chip according to <12>, wherein the lid includes a reflection material, a seal material containing a reflection layer, or a plate material containing a reflection layer.
<15> A target substance detection device, including:
   the target substance detection chip according to any one of <1> to <14>;
   a light irradiation unit configured to irradiate the electric field enhancement layer with light from a side of a surface of the target substance detection chip opposite to a surface of the target substance detection chip in which a flow path is formed; and
   a light detection unit configured to detect light reflected from the electric field enhancement layer.
<16> A target substance detection device, including:
   the target substance detection chip according to any one of <1> to <14>;
   a light irradiation unit configured to irradiate the electric field enhancement layer with light from a side of a surface of the target substance detection chip opposite to a surface of the target substance detection chip in which a flow path is formed; and
   a light detection unit configured to detect fluorescence emitted from the target substance or a fluorescent substance labeling the target substance in the analyte liquid present in the flow path, based on the irradiation with the light.
<17> The target substance detection device according to <15> or <16>, wherein the light irradiation unit includes:
   a light source; and
   a polarizing plate configured to polarize light emitted from the light source into linearly polarized light.
<18> A target substance detection method for detecting a target substance using the target substance detection device according to <15>, the method including:
   delivering the analyte liquid verifying a presence of the target substance through the flow path in the target substance detection chip;
   irradiating the electric field enhancement layer with light from a side of a surface of the target substance detection chip opposite to a surface of the target substance detection chip in which the flow path is formed; and
   detecting light reflected from the electric field enhancement layer.
<19> A target substance detection method for detecting a target substance using the target substance detection device according to <16>, the method including:
   delivering the analyte liquid verifying a presence of the target substance through the flow path in the target substance detection chip;
   irradiating the electric field enhancement layer with light from a side of a surface of the target substance detection chip opposite to a surface of the target substance detection chip in which the flow path is formed; and
   detecting fluorescence emitted from the target substance or a fluorescent substance labeling the target substance in the analyte liquid present in the flow path, based on the irradiation with the light.
<20> A target substance detection plate, including:
   a translucent plate main body in which one or more accommodation units and a flow path are formed, the accommodation unit having a shape of a recess each accommodating the target substance detection chip according to any one of <1> to <11> which detects a target substance, the flow path allowing the analyte liquid verifying a presence of the target substance to be delivered to the accommodation unit; and
   the target substance detection chip accommodated in the accommodation unit,
   wherein the flow path in the target substance detection chip is connected to the flow path in the plate main body to form a detection groove into which the analyte liquid is introduced.
<21> The target substance detection plate according to <20>, wherein the plate main body includes a disc-like member.
<22> The target substance detection plate according to <20> or <21>, wherein the plate main body is formed of a disc-like member and includes:
   an analyte liquid storage unit configured to store the analyte liquid and a cleaning fluid storage unit configured to store a cleaning fluid, the analyte liquid storage unit and the cleaning fluid storage unit being disposed at positions closer to a center of a circle of the disc-like member than the accommodation unit; and
   a waste liquid storage unit disposed at a position farther from the center of the circle than the accommodation unit and configured to store a waste liquid including the analyte liquid and the cleaning fluid, and
   each of the analyte liquid storage unit, the cleaning fluid storage unit, and the waste liquid storage unit is connected to the accommodation unit via the flow path in the plate main body through which the analyte liquid, the cleaning fluid, and the waste liquid are delivered.
<23> The target substance detection plate according to any one of <20> to <22>, wherein the detection groove appears in cross section to be shaped like a trapezoid.
<24> The target substance detection plate according to <23>, wherein a light blocking portion is formed on a bottom surface of the detection groove.
<25> The target substance detection plate according to any one of <20> to <24>, wherein a plurality of detection grooves is formed in parallel with respect to one target substance detection chip.
<26> The target substance detection plate according to <25>, wherein a spacing is provided between groove portions of the adjacent detection grooves.
<27> The target substance detection plate according to <26>, wherein the light blocking portion is formed in an area forming the spacing between the groove portions.
<28> A target substance detection device, including:
   the target substance detection plate according to any one of <20> to <27>;
   a light irradiation unit configured to irradiate the electric field enhancement layer with light from a side of a surface of the target substance detection chip opposite to a surface of the target substance detection chip in which the detection groove is formed; and
   a light detection unit configured to detect fluorescence emitted from the target substance or a fluorescent substance labeling the target substance in the analyte liquid present in the detection groove, based on the irradiation with the light.
<29> A target substance detection method for detecting a target substance using the target substance detection device according to <28>, the method including:
   delivering the analyte liquid through the flow path in the plate main body of the target substance detection plate to introduce the analyte liquid into the detection groove in the target substance detection chip;
   irradiating the electric field enhancement layer with light from a side of a surface of the target substance detection chip opposite to a surface of the target substance detection chip in which the detection groove is formed; and
   detecting fluorescence emitted from the target substance or a fluorescent substance labeling the target substance in the analyte liquid present in the detection groove, based on the irradiation with the light.

### Advantageous Effects of Invention

The present invention can provide a target substance detection chip, a target substance detection device, and a target substance detection method, that can solve the various problems in the conventional art described above, that can be manufactured easily in a small size at low costs with reduction of the number of parts involved in the detection chip constituted by an optical prism and a detection plate used for a SPR sensor and an optical waveguide mode sensor, that can detect a target substance quickly with high sensitivity, and in which an analyte liquid is easily delivered.

The present invention can also provide a target substance detection plate, target substance detection device, and a target substance detection method, that can be manufactured easily in a small size at low costs with reduction of the number of parts involved in a detection chip constituted by an optical prism and a detection plate used for a SPR sensor and an optical waveguide mode sensor, that can detect a target substance quickly with high sensitivity, that includes a detection chip to which an analyte liquid is easily introduced, and that can measure a target substance efficiently.

### Brief Description of Drawings

Fig. 1 is an explanatory diagram showing an example optical arrangement of a SPR sensor according to a conventional technique utilizing a surface plasmon resonance.
Fig. 2 is an explanatory diagram showing another example optical arrangement of a SPR sensor according to a conventional technique utilizing a surface plasmon resonance.
Fig. 3 is an explanatory diagram showing an example optical arrangement of an optical waveguide mode sensor according to a conventional technique.
Fig. 4 is an explanatory diagram showing another example optical arrangement of an optical waveguide mode sensor according to a conventional technique.
Fig. 5A is a perspective diagram showing a target substance detection chip according to an embodiment of the present invention.
Fig. 5B is a side elevation of the target substance detection chip shown in Fig. 5A.
Fig. 5C is an explanatory diagram of the target substance detection chip shown in Fig. 5B.
Fig. 6A is plan view of a target substance detection chip according to another embodiment of the present invention.
Fig. 6B is a cross-sectional diagram of Fig. 6A taken along a line A-A.
Fig. 6C is a cross-sectional diagram of Fig. 6A taken along a line B-B.
Fig. 7A is a cross-sectional diagram (1) showing a state that a lid is provided.
Fig. 7B is a cross-sectional diagram (2) showing a state that a lid is provided.
Fig. 8A is a cross-sectional diagram of a target substance diction chip according to still another embodiment of the present invention.
Fig. 8B is a cross-sectional diagram of a target substance detection chip according to still another embodiment of the present invention.
Fig. 9 is an explanatory diagram showing a target substance detection device according to an embodiment of the present invention,
Fig. 10 is a cross-sectional diagram of a target substance detection chip according to still another embodiment of the present invention.
Fig. 11 is a cross-sectional diagram of a target substance detection chip according to still another embodiment of the present invention.
Fig. 12 is a cross-sectional diagram of a target substance detection chip according to still another embodiment of the present invention.
Fig. 13 is a cross-sectional diagram of a target substance detection chip according to still another embodiment of the present invention.
Fig. 14 is an explanatory diagram showing a target substance detection device according to another embodiment of the present invention.
Fig. 15 is an explanatory diagram explaining a target substance detection plate according to an embodiment of the present invention.
Fig. 16 is an explanatory diagram explaining part of a target substance detection plate according to an embodiment of the present invention.
Fig. 17A is a diagram equivalent to a cross-sectional diagram of Fig. 16 taken along a line A-A.
Fig. 17B is a diagram equivalent to a cross-sectional diagram of Fig. 16 taken along a line B-B.
Fig. 18A is a plan view of a target substance detection plate according to another embodiment of the present invention.
Fig. 18B is an explanatory diagram explaining part of a target substance detection plate according to another embodiment of the present invention.
Fig. 19A is a perspective diagram showing a target substance detection chip according to the invention,.
Fig. 19B is a side elevation of the target substance detection chip shown in Fig. 19A.
Fig. 19C is an explanatory diagram showing the target substance detection chip shown in Fig. 19B.
Fig. 20 is a cross-sectional diagram showing another example target substance detection chip.
Fig. 21 is a cross-sectional diagram showing still another example target substance detection chip.
Fig. 22 is a cross-sectional diagram showing still another example target substance detection chip.
Fig. 23 is a cross-sectional diagram showing still another example target substance detection chip.
Fig. 24 is an explanatory diagram explaining part of an optical arrangement of a target substance detection device according to an embodiment of the present invention.
Fig. 25 is an explanatory diagram showing a target substance detection device according to an example of the present invention.
Fig. 26 is a graph showing the dependency on the wavelength, of the intensity of light transmitted as a result of irradiation of p-polarized linearly polarized light and s-polarized linearly polarized light.
Fig. 27 is a photograph showing an example result of detection of a target substance with a target substance detection device according to an example of the present invention.
Fig. 28 is an explanatory diagram showing an optical system manufactured for confirmation of effectiveness of a target substance detection chip.
Fig. 29 is a graph showing the dependency on the wavelength, of the intensity of light transmitted as a result of irradiation of p-polarized linearly polarized light and s-polarized linearly polarized light.

### Description of Embodiments

### (Target Substance Detection Device and Target Substance Detection Chip)

First, a first embodiment including a target substance detection chip of the present invention will be explained.

A target substance detection device of the present invention includes the target substance detection chip of the present invention, a light irradiation unit, a light detection unit, and according to necessity, other members.

The target substance detection device of the present invention can detect, for example, biomaterials such as viruses, proteins, and DNAs, heavy metals, oils, poisonous substances, deleterious substances, and various molecules as target substances. It can also observe changes in the nature of a substance that are accompanied by changes in dielectric constant.

### <Target substance Detection Chip>

The target substance detection chip includes a transparent base portion, a flow path, and according to necessity, other members.

### -Transparent Base Portion-

The transparent base portion is configured as a light-transmissive plate-like member.

The material from which the transparent base portion is made is not particularly limited and can be appropriately selected according to the purpose, as long as the material has the light transmissivity and allows formation of the flow path. Preferable examples thereof include plastic materials such as polystyrene and polycarbonate that can be mass-manufactured with injection molding techniques, and glass materials such as silica glass with which high transparency can be ensured.

The transparent base portion has a function of an optical prism used in conventional SPR sensors or optical waveguide mode sensors.

That is, it has a function of introducing light from the light irradiation unit into inclined surfaces of a later-described groove portion formed in the transparent base portion, at a specific incident angle at which a surface plasmon resonance or an optical waveguide mode will be excited.

Therefore, the lower limit of the refractive index of the transparent base portion is preferably 1.33 or greater, more preferably 1.38 or greater, and still more preferably 1.42 or greater.

The upper limit of the refractive index is preferably 4 or less, and more preferably 3 or less.

The refractive index will be described later with reference to the drawings.

### -Flow Path-

The flow path is formed in a surface of the transparent base portion as a groove, and an analyte liquid verifying the presence of the target substance is delivered through the groove in a length direction of the groove. Furthermore, the flow path is formed such that at least an electric field enhancement layer is disposed on an inner surface of a groove portion formed to at least partly have inclined surfaces appearing in cross section to be inclined at a gradient to the surface of the transparent base portion. Here, the electric field enhancement layer refers to a layer (surface plasmon excitation layer) formed to have a layered structure that enables surface plasmon resonance to be excited and a layer formed to have a layered structure that enables an optical waveguide mode to be excited. The electric field enhancement layer will be separately described below. Additionally, in the flow path, a part or entirety of an uppermost surface of the groove which contacts the analyte liquid forms a detection surface for the target substance.

Such a flow path configuration can serve both as a delivery path through which the analyte liquid is delivered and as a detection surface that detects the target substance. The flow path configuration can thus easily deliver the analyte liquid and enables a more significant reduction in production costs than a configuration in which the delivery path and the detection surface are separately formed.

The number of the flow paths in the target substance detection chip is not particularly limited and can be appropriately selected according to the purpose. One or more flow paths may be provided.

The shape of the flow path in a direction in which the analyte liquid flows is not particularly limited and can be appropriately selected according to the purpose. The shape may be linear or curved. However, when irradiated light is linearly polarized, p-polarized light is preferably constantly incident on the inclined surfaces in order to efficiently excite the surface plasmon resonance. Furthermore, preferably, s-polarized light or p-polarized light is constantly incident on the inclined surfaces in order to efficiently excite the optical waveguide mode. Thus, preferably, a portion of the flow path which is used for detection is linear.

Furthermore, the groove in the flow path is formed such that the electric field enhancement layer is disposed in the groove portion formed in the transparent base portion. Thus, the groove has a sectional shape similar to the shape of the groove portion.

### --Groove Portion--

A method for forming the groove portion in the transparent base portion is not particularly limited and can be appropriately selected according to the purpose. The method may be, e.g. a method for injection molding the transparent base portion so that the transparent base portion has the groove portion or a method for forming the groove portion in the transparent base portion using mechanical means, e.g., cutting means. Among these methods, the injection molding method is preferable because the method allows the target substance detection chip to be inexpensively and productively manufactured.

The groove portion at least partly has inclined surfaces appearing in cross section to be inclined at a gradient to a surface of the transparent base portion.

The shape of the groove portion is not particularly limited provided that the groove portion at least partly has the inclined surface. The shape may be, e.g., a cross-sectional V shape, a cross-sectional trapezoid, or a cross-sectional polygon. However, the shape does not include a cross-sectional U shape or a cross-sectional semi-circle, in which the inclined surfaces are curved surfaces and which has no portion inclined at the gradient. When the inclined surfaces are curved surfaces, the excitation of the surface plasmon or the optical waveguide mode in the electric field enhancement layer is limited. This precludes the target substance from being sufficiently detected.

Thus, groove side surfaces constituting the groove portion need to at least partly have inclined surfaces inclined at the gradient. On the other hand, in this view, the inclined surfaces may be formed into detection surfaces that detect the target substance and need not be formed all over the groove portion in a length direction thereof.

Furthermore, even when the inclined surfaces are formed all over the groove portion in the length direction thereof, not all of the inclined surfaces need to be used for detection. Detection may be performed by irradiating only a part of the inclined surfaces with light or capturing the target substance only on a part of the inclined surface.

The groove side surfaces constituting the groove portion are not particularly limited as long as the groove side surfaces have such an inclined surface. The groove side surfaces may be formed to be laterally symmetric or asymmetric.

This will be separately described below with reference to the drawings.

The opening width of the groove portion as viewed from above the surface of the transparent base portion, i.e., the spacing between the right and left side surfaces of the groove portion in the surface of the transparent base portion, is not particularly limited but is preferably 5 µm to 5 cm. When the opening width is less than 5 µm, the structure is very small, thus making production of the structure difficult and increasing manufacturing costs. Furthermore, a small size of the groove portion makes the flow path narrow, preventing a viscous liquid from flowing through the flow path. On the other hand, when the opening width is more than 5 cm, the internal volume of the flow path correspondingly increases, leading to the need for a large amount of analyte liquid.

Additionally, the depth of the groove portion is not particularly limited, but is preferably 5 µm to 5 cm for the same reason as that described above.

In addition, when a plurality of the flow paths is disposed, i.e., a plurality of the groove portions is formed, the spacing between the adjacent groove portions is not particularly limited but is preferably 5 µm to 5 cm. When the spacing is less than 5 µm, the structure is very small, thus making production of the structure difficult and increasing manufacturing costs. Furthermore, the small spacing is likely to cause the analyte liquid to leak between the adjacent flow paths, leading to possible mixture of the analyte liquid. When the spacing is more than 5 cm, the detection chip itself has an increased size, disadvantageously resulting in, e.g., the need for a larger amount of material for production and a large storage space.

### --Electric Field Enhancement Layer--

The electric field enhancement layer is not particularly limited and can be appropriately selected according to the purpose. The electric field enhancement layer may be formed, e.g., by (A) disposing a surface plasmon excitation layer inducing the surface plasmon resonance on the groove portion or by (B) disposing a layer structure exciting the optical waveguide mode on the groove portion. In this case, the layer structure exciting the optical waveguide mode can be formed by disposing a thin layer formed of a metal material or a semiconductor material and an optical waveguide layer formed of a transparent material, on the groove portion in this order.
(A) A formation material for the surface plasmon excitation layer is not particularly limited and can be appropriately selected according to the purpose. The formation material is, e.g., a metal material with a negative dielectric constant at the wavelength of incident light but is preferably a metal material containing at least one of gold, silver, platinum, and aluminum.

When a metal layer formed of the metal material receives light at a certain incident angle via a prism, an evanescent wave permeating toward a surface side of the metal layer meets excitation conditions for surface plasmon, inducing the surface plasmon resonance on the surface of the metal layer.

An optimum value for the thickness of the metal layer is determined by the metal material and the wavelength of the incident light. As is well known, the value can be calculated using Fresnel equations. In general, when the surface plasmon is excited in a near-ultraviolet to near-infrared region, the metal layer is several nm to several tens of nm in thickness.

A method for forming the surface plasmon excitation layer, i.e., the metal layer is not particularly limited but may be a well-known formation method, e.g., a vapor deposition method, sputtering method, a CVD method, a PVD method, or a spin coat method. However, when the formation material for the transparent base portion, in which the groove portion is formed, is the plastic material or the glass material, formation of the metal layer directly on the groove portion results in low adhesion, possibly causing the metal layer to be easily peeled off.

Thus, preferably, to improve the adhesion, an adhesion layer is formed on an inner surface of the groove portion using nickel or chromium as a formation material, with the metal layer formed on the adhesion layer.

If luminescence from the target substance or a fluorescent substance labeling the target substance is detected as described below, when the target substance or the fluorescent substance is in proximity to the metal layer, a phenomenon called quenching occurs in which emitted light is absorbed by the metal layer again to reduce luminous efficiency.

In this case, as is well-known, when a covering layer with a thickness of the order of several nm to several tens of nm is formed in order to separate the target substance and the fluorescent substance from the surface of the metal layer, quenching is inhibited to suppress a decrease in luminous efficiency.

Thus, the surface of the surface plasmon excitation layer, i.e., the metal layer is preferably covered with a transparent dielectric.

The transparent dielectric is not particularly limited but may be a material enabling formation of a transparent film with a thickness of several nm to several tens of nm, e.g., a glass material such as silica glass, an organic polymer material, or protein such as bovine serum albumin.
(B) In the formation of the layer structure that excites the optical waveguide mode, the metal material forming the thin layer is not particularly limited but may be, e.g., a generally available, stable metal or an alloy of the metal. Preferably, the metal material contains at least one of gold, silver, copper, platinum, and aluminum.

The semiconductor material forming the thin layer is not particularly limited but may be, e.g., a semiconductor material such as silicon or germanium or a known compound semiconductor material. In particular, silicon is preferable because this material is inexpensive and easy to process.

As is the case with the metal layer of the surface plasmon excitation layer, an optimum value for the thickness of the thin layer is determined by the material of the thin layer and the wavelength of the incident light, and as is well known, the value can be calculated using the Fresnel equations. In general, when light in a wavelength band in the near-ultraviolet to near-infrared region is used, the thin layer is several nm to several hundreds of nm in thickness.

When the metal layer is selected as the thin layer, the aforementioned adhesion layer formed of chromium or nickel is preferably disposed between the groove portion and the thin layer to improve the adhesion.

Furthermore, a formation material for the optical waveguide layer is not particularly limited provided that the formation material is transparent and has high light transmissivity. The formation material may be, e.g. silicon oxide, silicon nitride, a resin material such as an acrylic resin, a metal oxide such as titanium oxide, or a metal nitride such as aluminum nitride. Silicon oxide is preferable because this material is easy to produce and chemically stable. In this case, when the thin layer is formed of the silicon, the thin layer can be easily formed by oxidizing the surface side of the silicon layer.

### --Surface Treatment--

When the target substance is selectively detected, the surface of the flow path, i.e., the detection surface for the target substance, is preferably surface-treated so as to specifically capture the target substance, though the present invention is not limited to this surface treatment.

A method for the surface treatment is not particularly limited and can be appropriately selected according to the purpose. For example, when rare metal is used for the metal layer serving as the surface plasmon excitation layer to form the detection surface, a chemical modification method may be used in which a capturing substance is immobilized on the detection surface using metal-thiol bonding. Alternatively, when a glass material such as silica glass is used as the transparent dielectric covering the metal layer and this glass covering layer is used as the detection surface, a chemical modification method may be used in which the capturing substance is immobilized on the detection surface using silane coupling.

Furthermore, the surface treatment method carried out when a surface of the optical waveguide layer is used as the detection surface is not particularly limited and can be appropriately selected according to the purpose. For example, when silicon oxide is used as the optical waveguide layer and the surface of the optical waveguide layer is used as the detection surface, the chemical modification method may be used in which the capturing substance is immobilized on the detection surface using silane coupling, as is the case with the glass covering layer.

### -Other Members-

The other members are not particularly limited and can be appropriately selected according to the purpose. The other members include, e.g., a lid and a through-hole.

The lid is disposed on the surface of the transparent base portion in which the flow path is formed, so as to block the opening of the flow path in order to prevent the analyte liquid introduced into the flow path from spilling out from the flow path.

A formation material for the lid is not particularly limited. However, when luminescence from the target substance or the fluorescent substance labeling the target substance is detected, the lid is preferably formed of a transparent material that allows the emitted light to pass through. Thus, the presence of the target substance can be sensitively detected based on the detection of the luminescence by the light detection unit.

Such a lid is formed of, e.g., one of a seal material and a plate material which is formed of a transparent resin material or a transparent glass material.

When reflected light reflected from the electric field enhancement layer is detected, the lid may be formed of, e.g., a reflection material, a seal material containing a reflection layer, or a plate material containing a reflection layer so that the reflected light is reflected to the transparent base portion side and propagates through the transparent base portion.

The through-hole is formed to penetrate the transparent base portion in order to introduce the analyte liquid into the flow path and to discharge the analyte liquid introduced into the flow path.

For the through-hole, the surface of the transparent base portion opposite to the surface of the transparent base portion in which the flow path is formed is drilled so as to form two through holes, and penetrating ends of the through-holes are connected to a start point and an end point, respectively, of the flow path in the direction in which the analyte liquid flows.

An example of an embodiment of the target substance detection chip will be described with reference to the drawings.

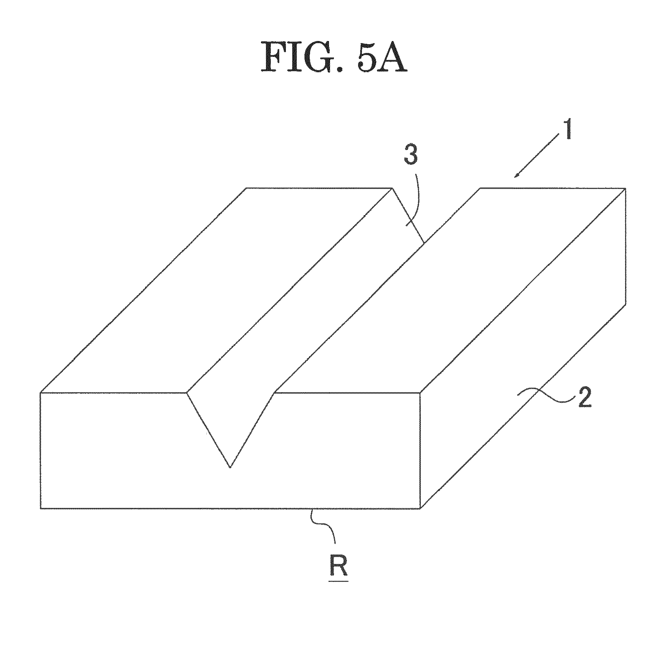

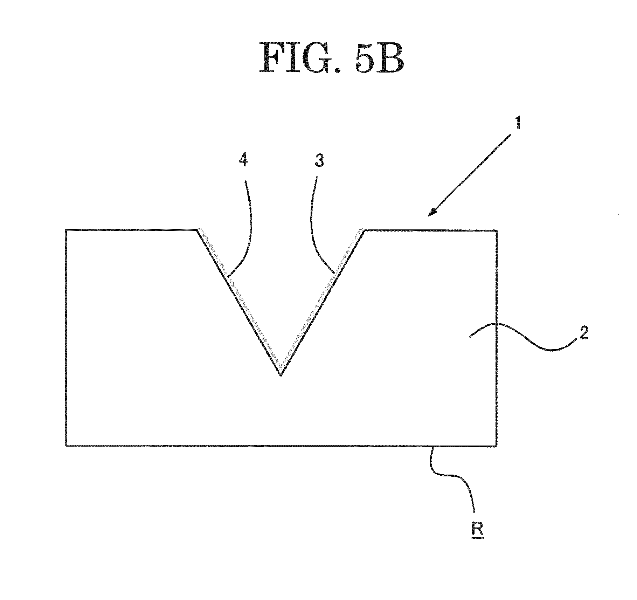

A target substance detection chip 1 according to an embodiment of the present invention shown in Fig. 5A has a configuration in which a flow path 3 consisting of a groove with a V-shaped cross section is formed in a surface of a transparent base portion 2. Furthermore, the flow path 3 is formed such that an electric field enhancement layer 4 is disposed on an inner surface of a groove portion formed to have a V-shaped cross section, as shown in Fig. 5B illustrating a cross section of the flow path 3. An analyte liquid that verifies the presence of a target substance is introduced into the flow path 3. The uppermost surface, in this case, a surface of the electric field enhancement layer 4, is used as a detection surface for the target substance.

In the electric field enhancement layer 4, light L irradiated from a light irradiation unit (not shown in the drawings) excites the surface plasmon resonance or the optical waveguide mode to form a strong electric field in the electric field enhancement layer 4 or near the surface of the electric field enhancement layer 4. The light irradiation unit irradiates the transparent base portion 2 with the light L from the side of a surface R of the transparent base portion 2 opposite to the surface of the transparent base portion 2 in which the flow path 3 is formed.

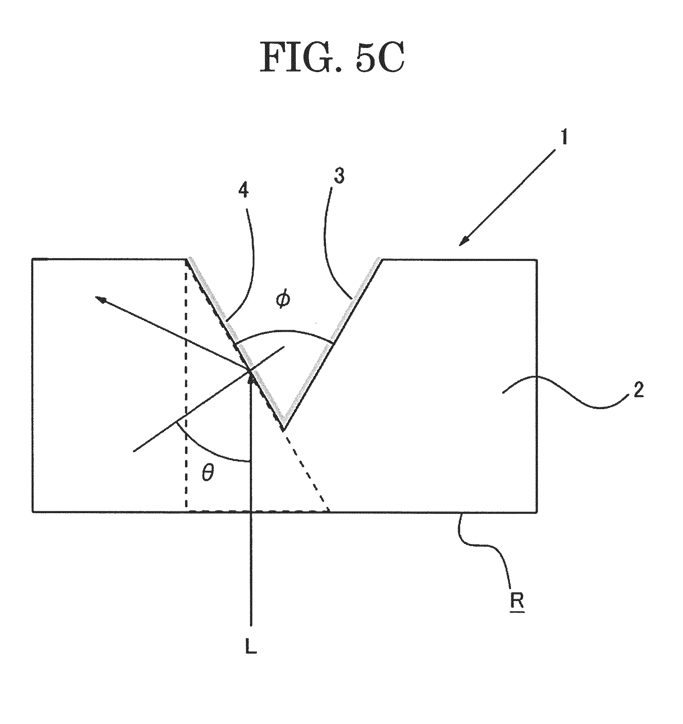

In this case, as shown in Fig. 5C illustrating Fig. 5B in detail, the transparent base portion 2 has an area shown by a dotted line and functioning as a triangular prism to allow the light L irradiated from the surface R side to enter the electric field enhancement layer 4 at a particular incident angle. That is, the transparent base portion 2 functions as an optical prism in an SPR sensor and an optical waveguide mode sensor to enhance the electric field in the vicinity of the surface of the electric field enhancement layer 4. This enables a target substance to be detected by this phenomenon. The surface R functions as an incident surface of the prism and thus preferably has high flatness.

The base angle φ of the groove portion of the transparent base portion 2 shown in Fig. 5C is determined by the incident angle θ of the light L to inclined surfaces forming the groove portion. For example, in the example of the target substance detection chip 1 shown in Fig. 5C, the surface of the transparent base portion 2 in which the flow path 3 is formed is parallel to the opposite surface R. Two inclined surfaces forming the groove portion are laterally symmetric. The light L from the light irradiation unit perpendicularly enters the surface R. In this case, the base angle φ (°) is selected such that φ = (90° - θ) × 2.

The incident angle θ is determined by excitation conditions for the surface plasmon resonance and the optical waveguide mode. Thus, the base angle φ depends on the refractive index of the formation material for the transparent base portion 2 and the configuration of the electric field enhancement layer 4.

In this case, an excessively low refractive index of the transparent base portion 2 results in the need to increase the incident angle θ and thus the need to reduce the base angle φ. When the flow path 3 is a micro flow path with an opening width of several hundred µm or less, a base angle φ of 30° or less makes formation of the flow path 3 difficult. Hence, the refractive index of the transparent base portion 2 is preferably 1.38 or greater and more preferably 1.42 or greater. On the other hand, when the size of the flow path does not particularly affect the degree of difficulty with which the flow path 3 is machined, the refractive index of the transparent base portion 2 may be lower but is preferably at least higher than the refractive index of water, 1.33.

On the other hand, an excessively high refractive index of the transparent base portion 2 disadvantageously causes the light L to be significantly reflected by the surface R when the light L enters the surface R. Furthermore, candidates for the material of the transparent base portion 2 are limited which have both a high refractive index and high transparency. Hence, the refractive index of the transparent base portion 2 is preferably 4 or less and more preferably 3 or less.

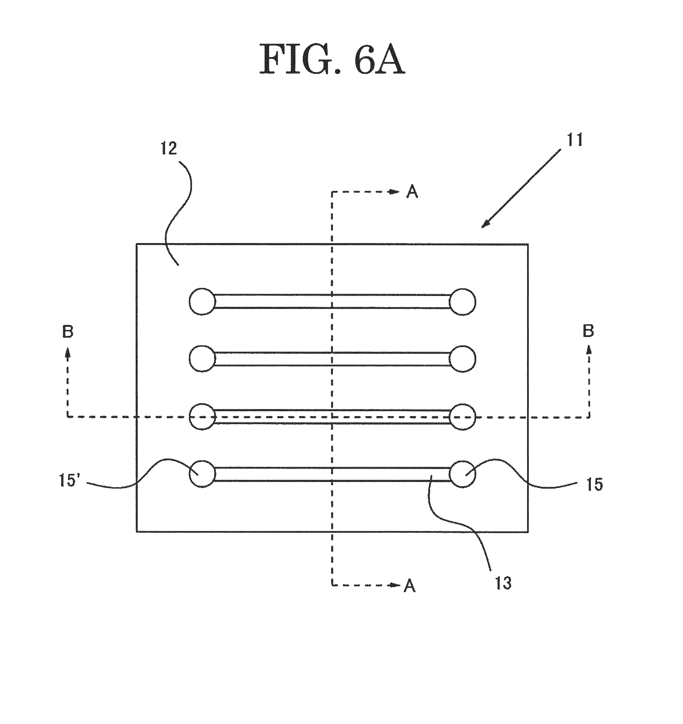

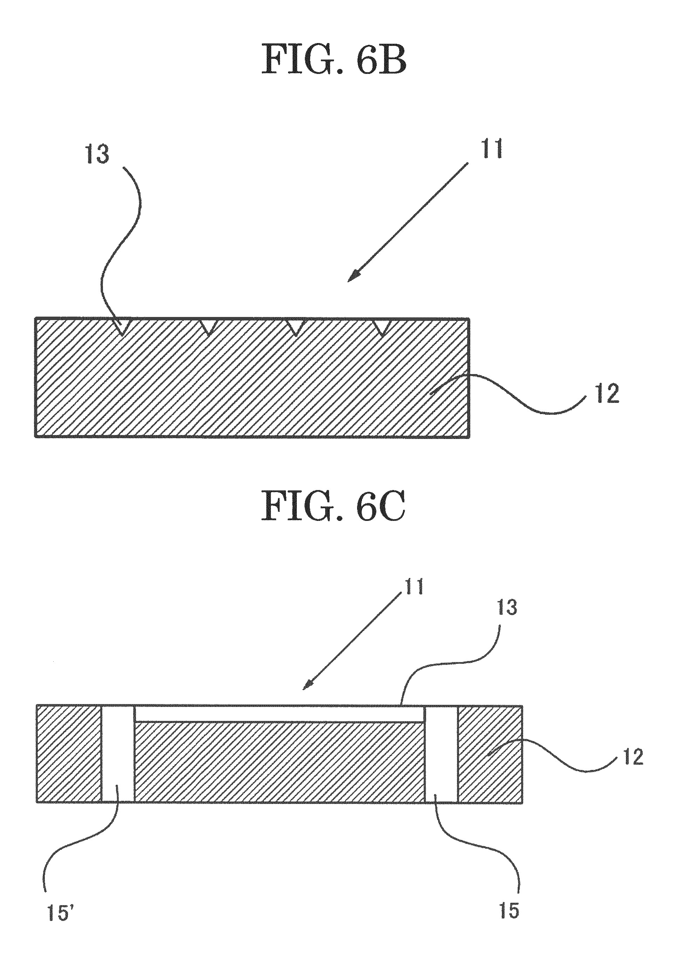

Another embodiment of the target substance detection chip will be described with reference to Fig. 6A. In a target substance detection chip 11 shown in Fig. 6A, four flow paths 13 are formed in a surface of a transparent base portion 12. Each of the flow paths 13 is formed to have a V-shaped cross section as a groove as shown in Fig. 6B showing a cross section taken along line A-A in Fig. 6A. The target substance detection chip 11 also includes a through-hole 15 formed therein and through which the analyte liquid is introduced into the flow path 13 and a through-hole 15' formed therein and through which the analyte liquid introduced into the flow path 13 is discharged, as shown in Fig. 6A and Fig. 6C showing a cross section taken along a line B-B in Fig. 6A,

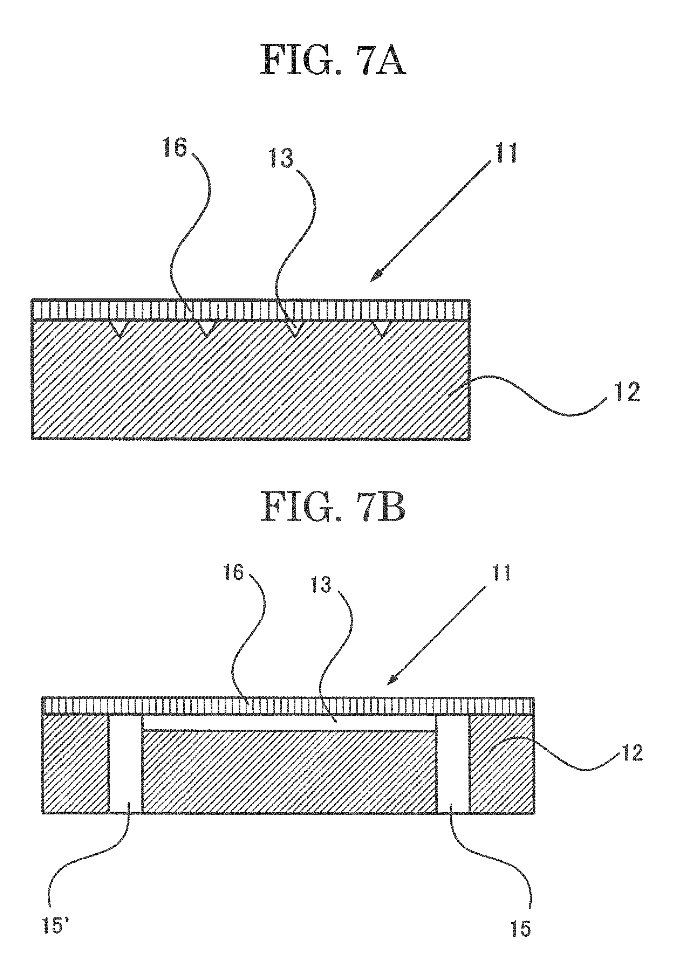

Fig. 7A and Fig. 7B are diagrams showing that a lid 16 is disposed on the target substance detection chip 11. That is, the lid 16 consists of a plate-like member or a sheet-like member and is disposed on the transparent base portion 12 so as to block the openings of the flow paths 13 to prevent the analyte liquid from spilling out from the flow paths 13. In this case, the analyte liquid is introduced from a lower opening of the through-hole 15 in Fig. 7B, flows through the flow path 13, and is discharged from a lower opening of the through-hole 15'. When the flow path 13 is thin, the analyte liquid is naturally delivered by capillary action. However, pressure may be applied to allow efficient delivery.

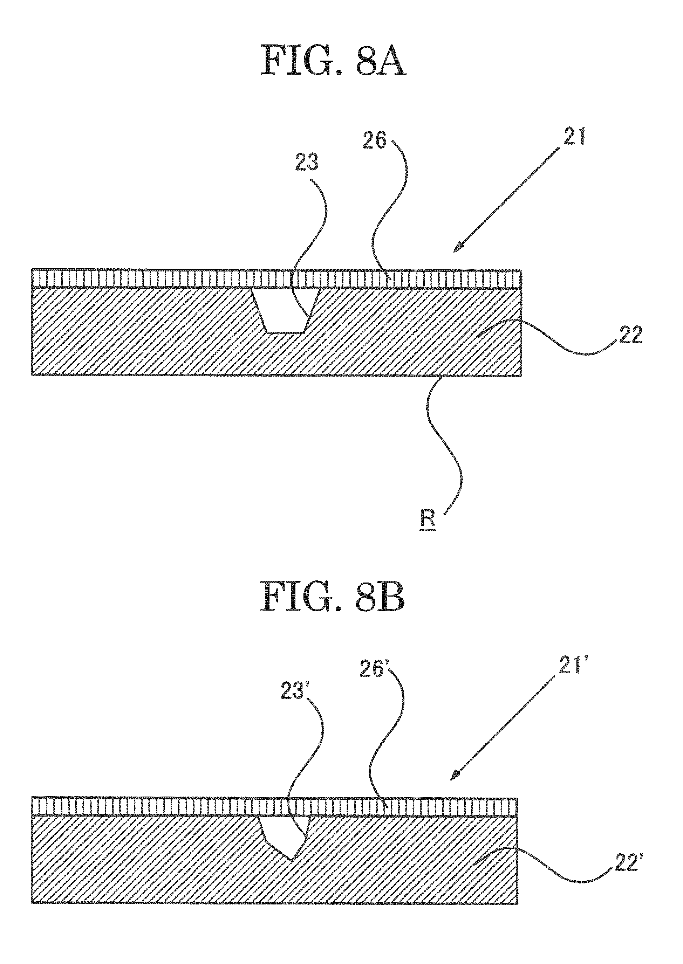

Fig. 8A is a cross-sectional view showing a modification in which the shape of the groove portion is changed from the V shape to a trapezoid. That is, a target substance detection chip 21 shown in Fig. 8A is constituted by a transparent base portion 22, a flow path 23, and a lid 26. The flow path 23 is formed to be trapezoidal.

When the groove portion and thus the flow path have a V shape, all the groove side surfaces of the flow path can advantageously be utilized as detection surfaces. On the other hand, a bottom portion of the flow path where the right and left groove side surfaces intersect subtends an acute angle, making difficult the removal, by cleaning, of the analyte liquid delivered to this portion and the target substance and other impurities captured in this portion. Thus, a heavy burden is imposed on the cleaning carried out for each detection test. Furthermore, detection may be erroneous due to the presence of the analyte liquid, target substance, and other impurities failing to be completely removed by cleaning.

In this regard, the trapezoidal flow path 23 prevents a bottom portion thereof from subtending an acute angle, thus allowing the used analyte liquid and target substance to be easily removed by cleaning. The widthwise (the lateral direction of Fig. 8A) length of the base of the trapezoid of the flow path 23 is preferably 2 µm to 4 cm when the opening width of the flow path 23 is 5 µm to 5 cm.

Fig. 8B is a cross-sectional view showing a modification in which the shape of the groove of the flow path is changed to a polygon. That is, a target substance detection chip 21' shown in Fig. 8B is constituted by a transparent base portion 22', a flow path 23', and a lid 26'. Groove side surfaces of a groove portion of the transparent base portion 22' and thus the groove side surfaces of the flow path 23' are formed at multiple gradients. In this case, detection can be performed simultaneously at different excitation wavelengths for the respective gradients.

A further modification of the flow path 23' will be described along with a specific detection method carried out by the target substance detection device, with reference to the subsequent figures.

### <Light Irradiation Unit>

The light irradiation unit is a unit that irradiates the electric field enhancement layer with light from the side of the surface of the target substance detection chip opposite to the surface of the target substance detection chip in which the flow path is formed.

The configuration of the light irradiation unit is not particularly limited and can be appropriately selected according to the purpose. The light irradiation unit may be configured by appropriately selecting any of well-known optical members, e.g., light sources such as a laser, a white lamp, and an LED, a collimator that collimates light from the light source, a lens that condenses the light from the light source, and a polarizing plate that polarizes the light from the light source.

In particular, the light irradiation unit is preferably configured to have a polarizing plate that polarizes light emitted from the light source into linearly polarized light.

### <Light Detection Unit>

The light detection unit is configured as (C) a unit that detects reflected light reflected from the electric field enhancement layer. Furthermore, when serving as (D) a detection unit to detect fluorescence from the target substance or the fluorescent substance labeling the target substance, the light detection unit is configured as a unit that detects fluorescence emitted from the target substance in the analyte liquid present in the flow path or the fluorescent substance, as a result of light irradiated from the light irradiation unit. These two aspects differ in the optical arrangement of the light detection unit.

The configuration of the light detection unit is not particularly limited and can be appropriately selected according to the purpose. In the case of (C), the light detection unit may be configured by appropriately selecting any of well-known optical members such as photodetectors such as a CCD, a photodiode, and a photomultiplier which detect the reflected light, an optical fiber that directs the reflected light to the photodetector, and a condensing lens that condenses and directs the reflected light to the photodetector.

Furthermore, when the spectral measurement method is used, the light detection unit includes a spectroscope and a photodetector to measure the spectrum of the reflected light or the light detection unit measures the intensity of the reflected light in a certain wavelength region.

Additionally, in the case of (D), the light detection unit may be configured by appropriately selecting any of well-known optical members such as photodetectors such as a CCD, a photodiode, or a photomultiplier which detect the fluorescence, an optical fiber that directs the fluorescence to the photodetector, and a condensing lens that condenses and directs the fluorescence to the photodetector. To determine whether the detected light is derived from the fluorescence emitted from the target substance or the fluorescent substance or from any other light, the photodetector may carry out detection via a wavelength filter that allows only light in the fluorescent wavelength band to pass through.

### <Other members>

The other members are not particularly limited and can be appropriately selected according to the purpose. The other members may be, e.g., a connecting flow path and a liquid delivery pump.

The connecting flow path consists of a flow path with any of various functions for any purpose and has, e.g., a branch portion that separates the analyte liquid and a junction portion that mixes the analyte liquid. The branch portion and the junction portion are arranged to connect to the above-described flow path or through-hole.

Furthermore, the liquid delivery pump may be a pump that delivers the analyte liquid to the flow path.

A specific example of a configuration in which the target substance detection device detects the target substance will be described below with reference to the drawings.

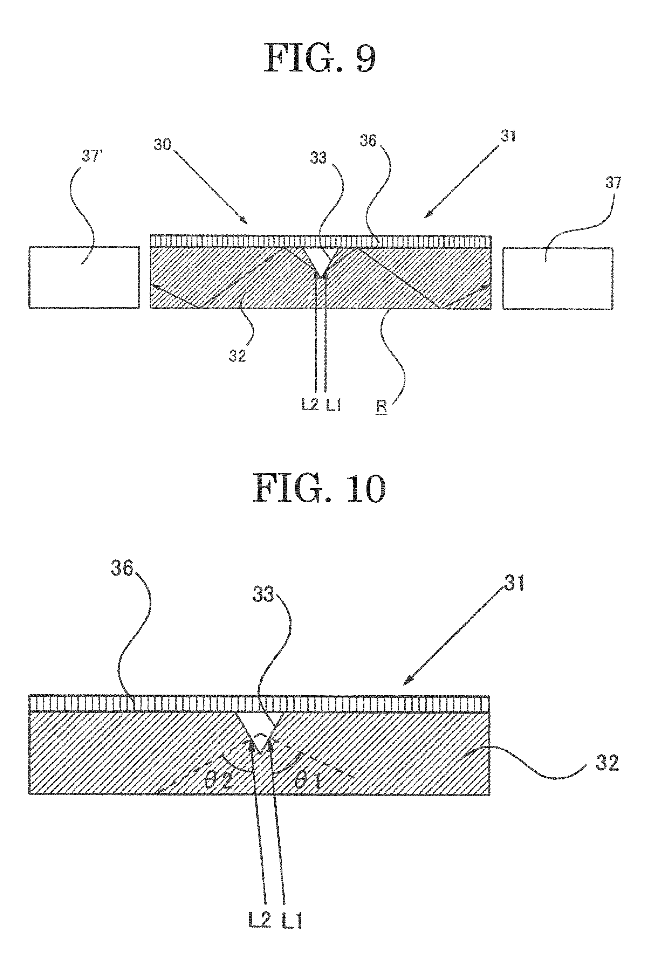

When the target substance is detected by detecting reflected light reflected from the electric field enhancement layer, the optical arrangement may be as shown in Fig. 9.

That is, a target substance detection device 30 is constituted by a target substance detection chip 31, a light irradiation unit (not shown in the drawings) that irradiates the target substance detection chip 31 with lights L1 and L2 from a surface R side, and two photodetectors 37 and 37' disposed in the vicinity of the respective lateral positions of the target substance detection chip 31. The target substance detection chip 31 is constituted by a transparent base portion 32, a flow path 33 in which right and left groove side surfaces constituting a groove portion of the transparent base portion 32 are laterally symmetric, and a lid 36 formed on the transparent base portion 32 so as to block the opening of the flow path 33.

The lights L1 and L2 irradiated from the surface R side of the target substance detection chip 31 to the flow path 33 are reflected in the lateral direction of Fig. 9 by two inclined surfaces of the flow path 33. In this case, the transparent base portion 32 is formed of a material having a higher refractive index than air, while the lid 36 is formed of a material having a lower refractive index than a formation material for the transparent base portion 32. Thus, reflected lights propagate through the transparent base portion 32 while being totally reflected. Furthermore, similar effects can be produced even when the lid 36 is formed of a reflection member.

When the transparent base portion 32 and the lid 36 have the same refractive index or the lid 36 has a higher refractive index than the transparent base portion 32, the reflected lights propagate through the transparent base portion 32 after being reflected from an upper surface of the lid 36.

The propagating lights exit from side surfaces of the transparent base portion 32, and thus, photodetectors 37 and 37' are arranged at the corresponding positions to detect the lights. In this case, to efficiently let the lights into the photodetectors 37 and 37', the side surfaces (light exit faces) of the transparent base portion 32 are preferably formed to be flat and thus polished as necessary. Furthermore, a condensing lens is preferably disposed near each of the light exit faces to let more light into the corresponding photodetector.

When monochromatic light such as laser light is used as the irradiated light, the presence or absence of the target substance is detected as follows. A mechanism for changing the incident angle is used to allow the light irradiation unit to rotate circumferentially in a semi-circle on the surface R side of the target substance detection chip 31 or to allow the target substance detection chip 31 to rotate circumferentially around the fixed light irradiation unit, to change the incident angle. During the circumferential rotation, a change in reflectance associated with excitation of the surface plasmon resonance or the optical waveguide mode is observed to determine a change in the dependency of the reflectance on the incident angle which may be caused by the capture of the target substance. In this regard, similar measurement may be carried out by angling light while condensing the light on the inclined surfaces of the flow path 33 by the condensing lens (not shown in the drawings), and observing a change in the reflection property associated with excitation of the surface plasmon resonance or the optical waveguide mode.

The change mechanism for changing the incident angle and a rotation mechanism for circumferentially rotating the target substance detection chip 31 need a movable portion and thus disadvantageously increase the size of the detection device itself. Thus, another preferable technique is to observe the intensity of the reflected light with the incident angle fixed to a given value to observe an increase and a decrease in the intensity of the reflected light which may be caused by the capture of the target substance and detect the target substance. This eliminates the need for a mechanism for condensing light on the inclined surfaces of the flow path 33.

When white light such as light from a lamp or an LED is used as the irradiated light, lights are collimated and then allowed to enter the target substance detection chip 31 from the surface R side thereof. The reflected lights are detected by the detectors 37 and 37' with the spectroscopes. The presence or absence of the target substance is detected by observing a reflection spectrum associated with excitation of the surface plasmon resonance or the optical waveguide mode to determine a change in reflection spectrum which may be caused by the capture of the target substance. Preferably, this configuration also eliminates the need for the change mechanism for changing the incident angle and a mechanism for condensing light on the inclined surfaces of the flow path 33, which are needed for the use of monochromatic light, thus allowing the device to be simplified.

When the white light is used, a change in light incident angle during measurement changes the dependency of the reflectance on the wavelength, thus making difficult reading of a change in reflection property due to the capture of the target substance.

Thus, in this case, the light incident angle is preferably fixed to a given value. The angle is not particularly limited and can be appropriately selected according to the purpose. For example, when the two inclined surfaces constituting the flow path 33 are laterally symmetric, lights perpendicularly entering the surface R enable the laterally arranged photodetectors 37 and 37' to detect the target substance.

Moreover, at this time, when the incident angle is changed with respect to the surface R as shown in Fig. 10, lights enter the right and left inclined surfaces at incident angles θ1 and θ2, and different reflection properties are obtained from the right and left inclined surfaces. Thus, different detections can be simultaneously carried out on the right and left inclined surfaces for any purpose.

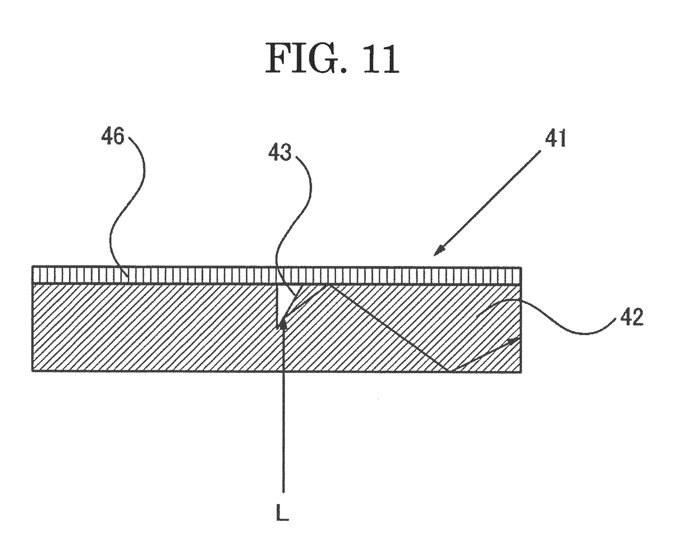

However, not both the right and left surfaces of the target substance detection chip 31 need be used for the above-described detection. The photodetector 37 may be disposed exclusively on one of the right and left of the target substance detection chip 31 for detection. Furthermore, the two inclined surfaces constituting the flow path 33 need not necessarily be laterally symmetric. For example, when the reflected light is detected on only one side of a target substance detection chip 41 as shown in Fig. 11, the angle of a surface on a side not used for detection does not particularly affect the detection, and may thus be in any form. For example, the surface on the side of a flow path 43 which is not used for the detection may be formed to be perpendicular as shown in Fig. 11. Reference numeral 46 in Fig. 11 denotes a lid.

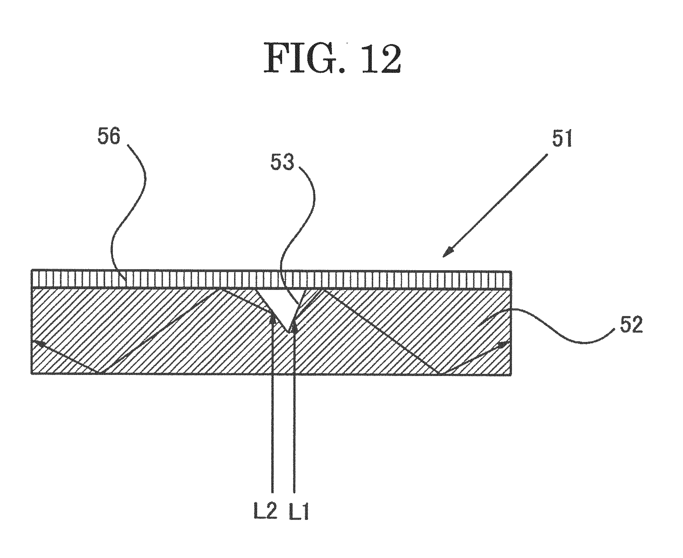

Furthermore, when two inclined surfaces constituting a groove portion in a transparent base portion 52 forming a flow path 53 are formed to be laterally asymmetric as shown in Fig. 12, different reflection properties are obtained from the respective inclined surfaces. Thus, two different detections can be carried out at the same time. In Fig. 12, reference numeral 51 denotes a target substance detection chip, and reference numeral 56 denotes a lid.

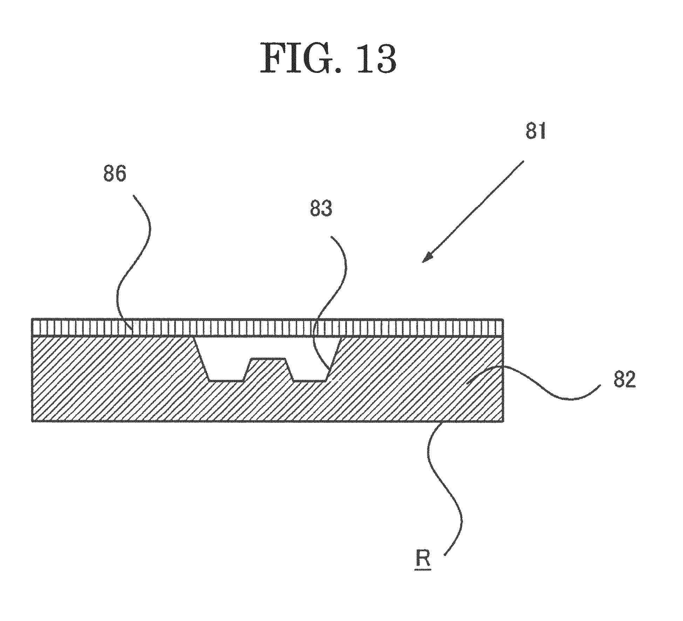

As shown in Fig. 13, when a groove portion of a transparent base portion 82 forming a flow path 83 is shaped like a polygon so as to provide a plane parallel to a surface R in the flow path 83, a bottom portion of the flow path 83 avoids subtending an acute angle, allowing the analyte liquid and the target substance to be easily removed by cleaning. Furthermore, when the thickness of the transparent base portion 82 is limited to preclude formation of a deep flow path, the use of the flow path 83 with a cross-sectional shape shown in Fig. 13 provides inclined surfaces larger than the inclined surfaces of the trapezoidal flow path shown in Fig. 8A. This allows higher sensitivity to be achieved. In this case, an upper side of a bottom protruding portion, that is, the portion between two inclined surfaces constituting the protruding portion, preferably has a certain width sufficient to prevent light reflected from one of the inclined surfaces constituting the protruding portion from being reflected again by the other of the inclined surfaces. In Fig. 13, reference numeral 81 denotes a target substance detection chip, and reference numeral 86 denotes a lid.

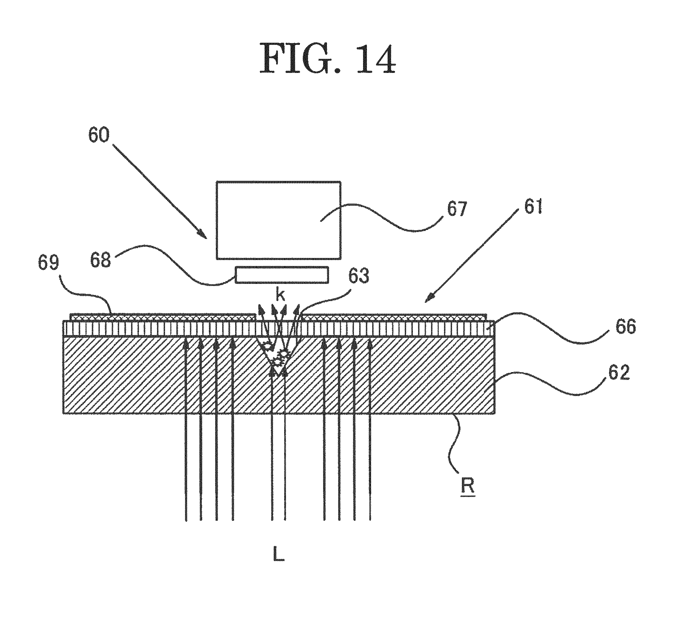

Fig. 14 shows an example of configuration of the target substance detection device for detecting fluorescence emitted from the target substance or the fluorescent substance. That is, a target substance detection device 60 has a target substance detection chip 61, a light irradiation unit (not shown in the drawings) that irradiates the target substance detection chip 61 with light L from a surface R side thereof, and a photodetector 67 that detects fluorescence k emitted from the target substance or the fluorescent substance, via a wavelength filter 68 that allows only light in the wavelength band of the fluorescence k to pass through. Furthermore, the target substance detection chip 61 has a transparent base portion 62, a flow path 63 formed in a surface of the transparent base portion 62, a lid 66 disposed on the transparent base portion 62 so as to block the flow path 63, and a light blocking portion 69 disposed at a position on the lid 66 other than a position opposite to the opening of the flow path 63. In Fig. 14, the light blocking portion 69 is formed on the lid 66, but may be formed between the lid 66 and the upper surface of the transparent base portion 62 other than a portion of the upper surface corresponding to the opening of the flow path 63.

In this case, the irradiated light L may be laser light corresponding to wavelengths in an excitation band for the target substance or the fluorochrome or light made monochromatic by an optical filter, a spectroscope, or the like. For the incident angle, light may enter the target substance detection chip 61 perpendicularly to or at a given angle to the surface R as is the case with the measurement of the reflected light.

In this case, the light irradiation unit is circumferentially rotated in a semi-circle on the surface R side of the target substance detection chip 61 or the target substance detection chip 61 is circumferentially rotated around the fixed light irradiation unit to change the incident angle of the light L to irradiate the electric field enhancement layer on the flow path 63 with the light L. Then, a phenomenon can be observed in which the luminous intensity increases at a particular angle at which the surface plasmon resonance or the optical waveguide mode is excited. This allows determination of whether the observed luminescence has resulted from the surface plasmon resonance or the optical waveguide mode or the fluorescent substance, upon being irradiated with a stray portion of the light L not involved in the excitation of the surface plasmon resonance or the optical waveguide mode, has emitted light independently of detection of the target substance.

However, as is the case with the detection of the reflected light, the change mechanism for changing the incident angle and the rotation mechanism for circumferentially rotating the target substance detection chip 61 need a movable portion. This may disadvantageously increase the size of the detection device itself. To allow a small, inexpensive device to be configured, a technique is preferably used in which the intensity of fluorescence is observed with the incident angle fixed to a given value to detect the target substance.

Even when fluorescence is detected, the target substance detection chip having a flow path with a groove structure similar to the groove structure in the above-described case where reflected light is detected may be used. That is, one of the following is applicable: a groove appearing to be V shaped in cross section as shown in Fig. 5B, a groove appearing to be trapezoidal in cross section as shown in Fig. 8A, a groove appearing to be polygonal in cross section as shown in Fig. 8B, a groove with only one of the inclined surfaces used for detection as shown in Fig. 11, a V-shaped groove that is laterally asymmetric as shown in Fig. 12, and the like. Furthermore, a groove appearing to be polygonal in cross section as shown in Fig. 13 is applicable. However, when planes parallel to the surface R are formed in the flow path as shown in Fig. 8A and as is the case of the target substance detection chip shown in Fig. 13, these planar portions are preferably provided with light blocking portions that attenuate light, so as to maximally prevent light from the light irradiation unit from reaching the photodetector side.

When the two inclined surfaces constituting the flow path 53 are formed to be laterally asymmetric as shown in Fig. 12, different fluorescent properties are obtained from the respective inclined surfaces. Thus, two different detections can be carried out at the same time.

For example, the right and left surfaces are set to induce electric field enhancement on the surfaces at different wavelengths. For example, the left surface is set such that the surface plasmon thereon is excited by 550-nm light. The right surface is set such that the surface plasmon thereon is excited by 660-nm light. The left surface is set to allow measurement of such an analyte as specifically adsorbs a fluorochrome that emits light when irradiated with 550-nm excitation light. The right surface is set to allow measurement of such an analyte as specifically adsorbs a fluorochrome that emits light when irradiated with 660-nm excitation light. Light sources are adapted to emit a 550-nm excitation light beam and a 660-nm excitation light beam, respectively. The light sources alternately irradiate the lights or the lights from the light sources are alternately blocked by filters or the like, to allow signals resulting from the excitation lights to be detected from the respective surfaces. Thus, two analytes can be detected at the same time. Moreover, when each of the two inclined surfaces constituting the flow path 23' is formed by a plurality of surfaces inclined at different angles as shown in Fig. 8B, detections at a larger number of different excitation wavelengths can be carried out at the same time.

When the target substance itself emits the fluorescence k, the presence or absence and the amount of the target substance can be observed by capturing the target substance on the detection surface of the flow path 63 and observing the presence or absence of luminescence from the target substance and the intensity of the luminescence.

However, many substances fail to exhibit a significant luminescence property. Thus, the target substance is captured on the detection surface of the flow path 63 and the fluorescent substance is attached to the target substance, and then the luminescence from the fluorescent substance is observed.

A method for attaching the fluorescent substance is not particularly limited, but a well-known technique is applicable. An exemplary method involves binding the fluorescent substance to an antibody specifically adsorbed by the target substance and allowing the antibody with the fluorescent substance to be adsorbed by the target substance.

### (Target Substance Detection Method)

One target substance detection method according to the present invention is a method for detecting the target substance using the target substance detection device according to the first embodiment of the present invention. The method includes an analyte liquid introduction step, a light irradiation step, and a light detection step.

The analyte liquid introduction step is a step of introducing an analyte liquid that verifies the presence of the target substance into the flow path in the target substance detection chip.

The light irradiation step is a step of irradiating the electric field enhancement layer with light from the side of the surface of the target substance detection chip opposite to the surface of the target substance detection chip in which the flow path is formed.

The light detection step is (E) a step of detecting light reflected from the electric field enhancement layer or (F) a step of detecting fluorescence emitted from the target substance in the analyte liquid present in the flow path or the fluorescent substance labeling the target substance, based on the irradiation with the light carried out in the light irradiation step.

These steps can be appropriately carried out based on the matters described for the target substance detection chip and the target substance detection device.

### (Target Substance Detection Device and Target Substance Detection Plate)

A second embodiment with a target substance detection plate according to the present invention will be described.

The target substance detection device according to the present invention has the target substance detection plate according to the present invention, a light irradiation unit, a light detection unit, and any other member as necessary.

The target substance detection device according to the present invention can detect, as a target substance, e.g., a biomaterial such as a virus, protein, DNA, or a biomarker, a contaminant, a poisonous substance, a deleterious substance, or any of various other molecules.

### <Target Substance Detection Plate>

The target substance detection plate has a translucent plate main body and a target substance detection chip that detects the target substance.

### -Plate Main Body-

The plate main body includes one or more accommodation units having a shape of a recess formed therein and each accommodating the target substance detection chip and flow paths formed therein and through which an analyte liquid verifying the presence of the target substance is delivered to the accommodation units.

The shape of the plate main body is not particularly limited and can be appropriately selected according to the purpose. For example, a disc-like plate member, a triangular plate-like plate member, or a rectangular plate-like plate member may be used.

A formation material for the plate main body is not particularly limited and can be appropriately selected according to the purpose provided that the formation material has translucency. The formation material may be, e.g. a plastic material such as cyclic polyolefin, acrylic, polystyrene, or polycarbonate, or a glass material that ensures high transmissivity.

A method for forming the accommodation unit is not particularly limited and can be appropriately selected according to the purpose. The method may be, e.g., a method for forming the plate main body by injection molding or a method for forming the accommodation unit by carrying out machining such as cutting on the plate main body.

The shape of the recess in the accommodation unit is not particularly limited but may be appropriately selected according to the shape and size of the accommodated target substance detection chip.

A bottom surface of the recess is preferably formed as a flat surface so as to stably contact a surface of the accommodated target substance detection chip.

A method for forming the flow path is not particularly limited and can be appropriately selected according to the purpose. The method may be, e.g., a method for forming the plate main body by injection molding or a method for forming the flow path by carrying out machining such as cutting on the plate main body.

The planar shape of the flow path appearing in a plan view of the plate main body is not particularly limited and can be appropriately selected according to the purpose. The planar shape may be, e.g., linear or curved. For example, when the plate main body is shaped like a disc, the flow path may have a shape curved along a direction in which the disc rotationally moves.

Furthermore, the cross-sectional shape of the flow path may be, e.g., a rectangle, a V shape, a semi-circle, a semi-ellipsoid, or a trapezoid.

The plate main body is not particularly limited but may further have an analyte liquid storage unit that stores the analyte liquid, a cleaning fluid storage unit that stores a cleaning fluid for removing the analyte liquid, and a waste liquid storage unit that stores a waste liquid containing the analyte liquid and the cleaning fluid. Furthermore, the plate main body may have a lid to prevent these liquids from spilling out. Additionally, to allow the liquids to smoothly enter these storage units, a vent hole is preferably formed to let out air in the storage units through the vent hole.

### -Target Substance Detection Chip-

The target substance detection chip according to the second embodiment may be configured substantially equivalently to the target substance detection chip described in the first embodiment. However, in the target substance detection chip according to the second embodiment, the flow path in the target substance detection chip described in the first embodiment is connected to the flow path in the plate main body to form a detection groove into which the analyte liquid is introduced. The target substance detection chip according to the second embodiment will be described below.

The target substance detection chip is accommodated in the accommodation unit and has a transparent base portion and a detection groove.

The target substance detection chip is accommodated in the accommodation unit so that a bottom surface of the accommodation unit is joined to a surface of the transparent base portion opposite to a surface of the transparent base portion in which the detection groove is disposed.

Furthermore, the target substance detection chip may be fixed to the accommodation unit or accommodated in the accommodation unit without being fixed.

When the target substance detection chip is not fixed, the position of the target substance detection chip is preferably regulated so as not to vary in the accommodation unit. The position is regulated, e.g., by forming the accommodation unit into a quadrangular prism or an elliptic cylinder by cutting and placing, inside the accommodation unit, the target substance detection chip shaped correspondingly like a quadrangular prism or an elliptic cylinder and which is slightly smaller than the accommodation unit.

### --Transparent Base Portion--

The transparent base portion is configured as a light-transmissive plate-like member.

A formation material for the transparent base portion is not particularly limited and can be appropriately selected according to the purpose provided that the formation material is light-transmissive and allows formation of the detection groove. Preferably, the formation material is, e.g., a plastic material such as polystyrene or polycarbonate which can be mass-manufactured using an injection molding technique or a glass material such as silica glass which can ensure high transparency.

The transparent base portion has a function of an optical prism used in conventional SPR sensors or optical waveguide mode sensors.

That is, the transparent base portion serves to introduce light irradiated from the light irradiation unit into inclined surfaces of a groove portion described below and formed in the detection groove, at a particular incident angle at which the surface plasmon resonance or the optical waveguide mode is excited.

Thus, the lower limit of the refractive index of the transparent base portion is preferably 1.33 or greater, more preferably 1.38 or greater, and most preferably 1.42 or greater. Furthermore, the upper limit of the refractive index is preferably 4 or less and more preferably 3 or less.

The refractive index will be separately described below with reference to the drawings.

The transparent base portion is disposed in the accommodation unit so that the surface of the transparent base portion opposite to the surface of the transparent base portion in which the detection groove is formed is in contact with or in proximity to a bottom portion of the accommodation unit. The opposite surface of the transparent base portion is used as a surface on which light irradiated from the bottom portion of the accommodation unit is incident, and is thus preferably formed to be flat.

### --Detection Groove--

The detection groove is formed in a surface of the transparent base portion and connected to the flow path in the plate main body so that the analyte liquid is introduced into the detection groove. Furthermore, the detection groove is formed such that an electric field enhancement layer is disposed on an inner surface of the groove portion formed to at least partly have inclined surfaces appearing in cross section to be inclined at a gradient to the surface of the transparent base portion. Here, the electric field enhancement layer refers to a layer (surface plasmon excitation layer) formed to have a layered structure that enables the surface plasmon resonance to be excited and a layer formed to have a layered structure that enables the optical waveguide mode to be excited. The electric field enhancement layer will be separately described below.

The number of the detection grooves is not particularly limited and can be appropriately selected according to the purpose. One or more detection grooves may be provided. However, the detection groove forms a detection surface that detects the target substance, and thus, the area of the detection groove is desirably increased as much as possible to improve detection sensitivity for the target substance. Therefore, a plurality of detection grooves is preferably provided.

In this case, a plurality of the detection grooves is preferably formed in parallel with respect to one target substance detection chip.

### ---Groove Portion---

A method for forming the groove portion is not particularly limited and can be appropriately selected according to the purpose. The method may be, e.g., a method for injection molding of the transparent base portion so that the transparent base portion has the groove portion or a method for forming the groove portion in the transparent base portion using mechanical means, e.g., cutting means.

The groove portion at least partly has the inclined surfaces appearing in cross section to be inclined at the gradient to a surface of the transparent base portion.

The shape of the groove portion is not particularly limited provided that the groove portion at least partly has the inclined surface. The shape may be, e.g., a cross-sectional V shape, a cross-sectional trapezoid, or a cross-sectional polygon. However, the shape does not include a cross-sectional U shape or a cross-sectional semi-circle, in which the inclined surfaces are curved surfaces and which has no portion inclined at the gradient. When the inclined surfaces are curved surfaces, the excitation of the surface plasmon or the optical waveguide mode in the electric field enhancement layer is limited. This precludes the target substance from being sufficiently detected.

Thus, groove side surfaces constituting the groove portion need to at least partly have inclined surfaces inclined at the gradient. On the other hand, in this view, the inclined surfaces may be formed into detection surfaces that detect the target substance and need not be formed all over the groove portion in a length direction thereof.

Furthermore, even when the inclined surfaces are formed all over the groove portion in the length direction thereof, not all of the inclined surfaces need to be used for detection. Detection may be performed by irradiating only a part of the inclined surfaces with light or capturing the target substance only on a part of the inclined surface.

The groove side surfaces constituting the groove portion are not particularly limited as long as the groove side surfaces have such an inclined surface. The groove side surfaces may be formed to be laterally symmetric or asymmetric.

This will be separately described below with reference to the drawings.

The opening width of the groove portion as viewed from above the surface of the transparent base portion, i.e., the spacing between the right and left side surfaces of the groove portion in the surface of the transparent base portion, is not particularly limited but is preferably 5 µm to 5 cm. When the opening width is less than 5 µm, the structure is very small, thus making production of the structure difficult and increasing manufacturing costs. Furthermore, a small size of the groove portion makes the detection groove narrow, preventing a viscous liquid from flowing through the detection groove. On the other hand, when the opening width is more than 5 cm, the internal volume of the detection groove correspondingly increases, leading to the need for a large amount of analyte liquid.

Additionally, the depth of the groove portion is not particularly limited, but is preferably 5 µm to 5 cm for the same reason as that described above.

In addition, when a plurality of the detection grooves is disposed, i.e., a plurality of the groove portions is formed, the spacing between the adjacent groove portions is not particularly limited but is preferably 5 µm to 5 cm. When the spacing is less than 5 µm, the structure is very small, thus making production of the structure difficult and increasing manufacturing costs. When the spacing is more than 5 cm, the target substance detection chip itself has an increased size, disadvantageously resulting in, e.g., the need for a larger amount of material for production and a large storage space.

In addition, light irradiated from the light irradiation unit passes directly through areas corresponding to the spacings between the groove portions, toward the light detection unit. Thus, a light blocking portion that attenuates light is preferably provided in these areas.

### ---Electric Field Enhancement Layer---

The electric field enhancement layer is not particularly limited and can be appropriately selected according to the purpose. The electric field enhancement layer may be formed, e.g., by (A) disposing a surface plasmon excitation layer inducing the surface plasmon resonance on the groove portion or by (B) disposing a layer structure exciting the optical waveguide mode on the groove portion. In this case, the layer structure exciting the optical waveguide mode can be formed by disposing a thin layer formed of a metal material or a semiconductor material and an optical waveguide layer formed of a transparent material, on the groove portion in this order.
(A) A formation material for the surface plasmon excitation layer is not particularly limited and can be appropriately selected according to the purpose. The formation material is, e.g., a metal material with a negative dielectric constant at the wavelength of incident light but is preferably a metal material containing at least one of gold, silver, platinum, and aluminum.

When a metal layer formed of the metal material receives light at a certain incident angle via a prism, an evanescent wave permeating toward a surface side of the metal layer meets excitation conditions for surface plasmon, inducing the surface plasmon resonance on the surface of the metal layer.

An optimum value for the thickness of the metal layer is determined by the metal material and the wavelength of the incident light. As is well known, the value can be calculated using the Fresnel equations. In general, when the surface plasmon is excited in the near-ultraviolet to near-infrared region, the metal layer is several nm to several tens of nm in thickness.

A method for forming the surface plasmon excitation layer, i.e., the metal layer is not particularly limited but may be a well-known formation method, e.g., a vapor deposition method, sputtering method, a CVD method, a PVD method, or a spin coat method. However, when the formation material for the transparent base portion, in which the groove portion is formed, is the plastic material or the glass material, formation of the metal layer directly on the groove portion results in low adhesion, possibly causing the metal layer to be easily peeled off.

Thus, preferably, to improve the adhesion, an adhesion layer is formed on an inner surface of the groove portion using nickel or chromium as a formation material, with the metal layer formed on the adhesion layer.

If luminescence from the target substance or a fluorescent substance labeling the target substance is detected as described below, when the target substance or the fluorescent substance is in proximity to the metal layer, a phenomenon called quenching occurs in which emitted light is absorbed by the metal layer again to reduce luminous efficiency.

In this case, as is well-known, when a covering layer with a thickness of the order of several nm to several tens of nm is formed in order to separate the target substance and the fluorescent substance from the surface of the metal layer, quenching is inhibited to suppress a decrease in luminous efficiency.

Thus, the surface of the surface plasmon excitation layer, i.e., the metal layer is preferably covered with a transparent dielectric.

The transparent dielectric is not particularly limited but may be a material enabling formation of a transparent film with a thickness of several nm to several tens of nm, e.g., a glass material such as silica glass, an organic polymer material, or protein such as bovine serum albumin.
(B) In the formation of the layer structure that excites the optical waveguide mode, the metal material forming the thin layer is not particularly limited but may be, e.g., a generally available, stable metal or an alloy of the metal. Preferably, the metal material contains at least one of gold, silver, copper, platinum, and aluminum.

The semiconductor material forming the thin layer is not particularly limited but may be, e.g., a semiconductor material such as silicon or germanium or a known compound semiconductor material. In particular, silicon is preferable because this material is inexpensive and easy to process.

As is the case with the metal layer of the surface plasmon excitation layer, an optimum value for the thickness of the thin layer is determined by the material of the thin layer and the wavelength of the incident light, and as is well known, the value can be calculated using the Fresnel equations. In general, when light in a wavelength band in the near-ultraviolet to near-infrared region is used, the thin layer is several nm to several hundreds of nm in thickness.

When the metal layer is selected as the thin layer, the aforementioned adhesion layer formed of chromium or nickel is preferably disposed between the groove portion and the thin layer to improve the adhesion.

Furthermore, a formation material for the optical waveguide layer is not particularly limited provided that the formation material is transparent and has high light transmissivity. The formation material may be, e.g. silicon oxide, silicon nitride, a resin material such as an acrylic resin, a metal oxide such as titanium oxide, or a metal nitride such as aluminum nitride. Silicon oxide is preferable because this material is easy to produce and chemically stable. In this case, when the thin layer is formed of the silicon, the thin layer can be easily formed by oxidizing the surface side of the silicon layer.

### ---Surface Treatment---

When the target substance is selectively detected, the surface of the detection groove, i.e., the detection surface, is preferably surface-treated so as to specifically capture the target substance, though the present invention is not limited to this surface treatment.

A method for the surface treatment is not particularly limited and can be appropriately selected according to the purpose. For example, when rare metal is used for the metal layer serving as the surface plasmon excitation layer to form the detection surface, a chemical modification method may be used in which a capturing substance is immobilized on the detection surface using metal-thiol bonding. Alternatively, when a glass material such as silica glass is used as the transparent dielectric covering the metal layer and this glass covering layer is used as the detection surface, a chemical modification method may be used in which the capturing substance is immobilized on the detection surface using silane coupling.

Furthermore, the surface treatment method carried out when a surface of the optical waveguide layer is used as the detection surface is not particularly limited and can be appropriately selected according to the purpose. For example, when silicon oxide is used as the optical waveguide layer and the surface of the optical waveguide layer is used as the detection surface, the chemical modification method may be used in which the capturing substance is immobilized on the detection surface using silane coupling, as is the case with the glass covering layer.

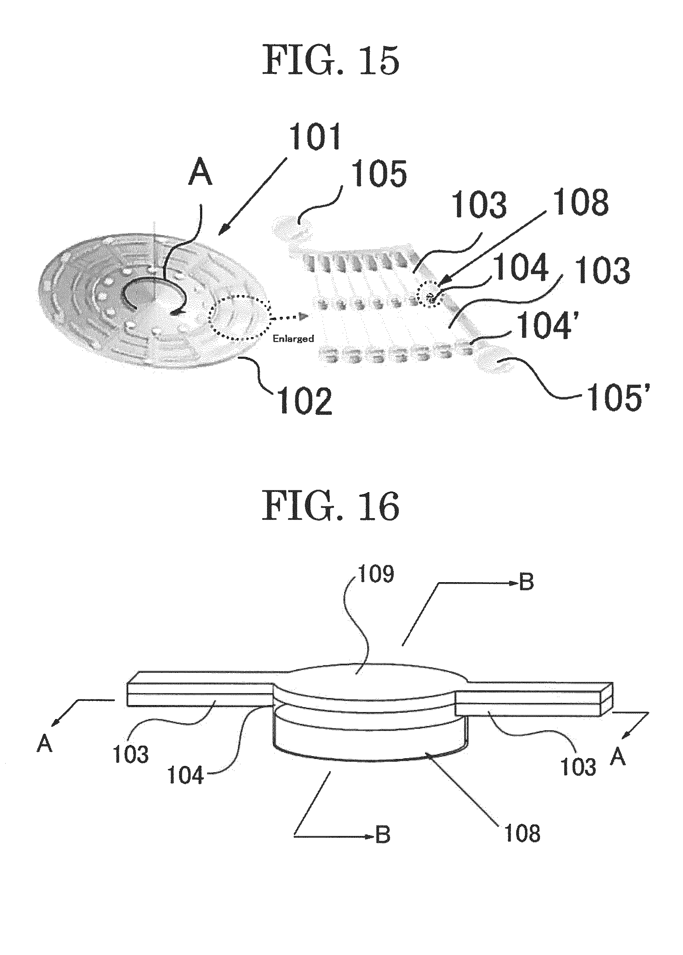

Now, an embodiment of the target substance detection plate will be described with reference to Fig. 15 and Fig. 16.

As shown in Fig. 15, a plate main body 102 of a target substance detection plate 101 is formed like a disc and can be rotationally moved in the direction of arrow A in Fig. 15 by a rotational movement unit such as a spindle (not shown in the drawings).

As shown in an enlarged portion of the plate main body 102, a flow path 103, an accommodation unit 104, and an analyte liquid storage unit 105 storing an analyte liquid are formed in the plate main body 102. Rotational movement of the plate main body 102 allows the analyte liquid to be introduced from the analyte liquid storage unit 105 into the accommodation unit 104 via the flow path 103. 104' and 105' denote waste liquid storage units for storing a waste liquid.

Furthermore, a target substance detection chip 108 is accommodated in the accommodation unit 104 to detect the target substance present in the analyte liquid. That is, as shown in Fig. 16, the accommodation unit 104 is formed like a recess in which the target substance detection chip 108 is accommodated. The accommodation unit 104 is connected at side surfaces thereof to the flow path 103 in the plate main body 102, thus enabling the analyte liquid to be delivered to the target substance detection chip 108. Reference numeral 109 in Fig. 16 is a lid disposed in order to prevent the analyte liquid from spilling out from the accommodation unit 104.

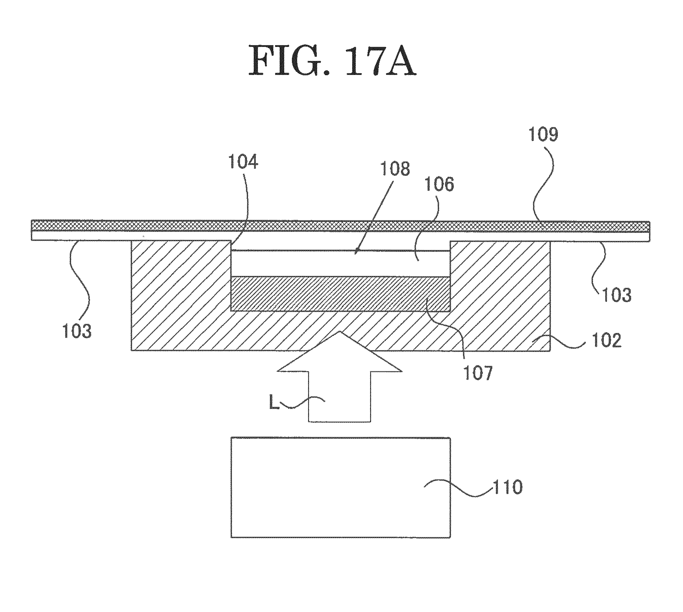

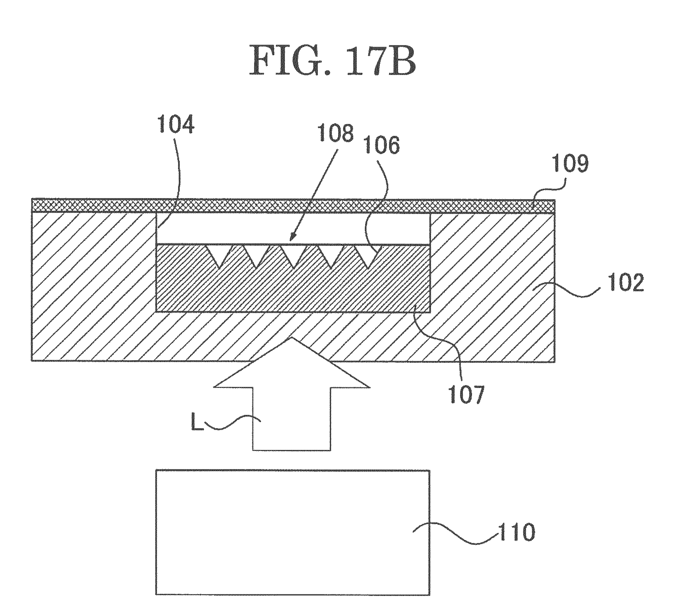

How the target substance detection chip 108 is accommodated in the accommodation unit 104 will be described with reference to Fig. 17A and Fig. 17B. Fig. 17A is a diagram corresponding to a cross section taken along line A-A in Fig. 16. Furthermore, Fig. 17B is a diagram corresponding to a cross section taken along line B-B in Fig. 16.

As shown in Figs. 17A and 17B, the target substance detection chip 108 is accommodated in the accommodation unit 104. The analyte liquid delivered through the flow path 103 in the plate main body 102 is introduced into a flow path in the target substance detection chip 108, i.e., a detection groove 106. A light source 110 installed outside the plate main body 102 irradiates a transparent base portion 107 with light L from the side of a surface of the transparent base portion 107 opposite to a surface of the transparent base portion 107 in which the detection groove 106 is formed. The light enters the detection groove 106. When the detection groove 106 is irradiated with the light, an electric field enhancement layer disposed in the detection groove 106 enhances an electric field, allowing fluorescence from the target substance or a fluorescent substance labeling the target substance to be observed. Detection of the target substance may be performed with the analyte liquid present on the detection groove 106. However, the detection is preferably carried out after a cleaning fluid is introduced into the accommodation unit 4 to clean the accommodation unit of impurities and contaminants after the target substance contained in the introduced analyte liquid is captured by a capturing substance immobilized on the detection surface. This is because signals from the impurities and contaminants can be excluded.

In this case, the target substance detection chip 108 is preferably arranged in the accommodation unit 104 so that the analyte liquid fed from a side of the flow path 103 in the plate main body 102 through which the analyte liquid is supplied to the accommodation unit 104 flows along the detection groove 106 in the target substance detection chip 108 and is then discharged to the flow path 103 joined to a waste liquid storage unit 105'. To implement this arrangement, preferably the detection groove 106 is disposed parallel to a straight line connecting an analyte liquid supply port leading to the accommodation unit 104, i.e., a junction between the accommodation unit 104 and the side of the flow path 103 through which the analyte liquid is supplied to the accommodation unit 104, to a discharge port through which the analyte liquid is discharged from the accommodation unit 104, i.e., a junction between the accommodation unit 104 and the flow path 103 through which a waste liquid is discharged from the accommodation unit 104, or a deviation from the parallel state is at an angle of ±45° or less. The thus connected flow path 103 and detection groove 106 allow the analyte liquid to be efficiently introduced from the flow path 103 into the detection groove 106. Furthermore, a cleaning fluid is easily introduced into the detection groove 106, allowing the analyte liquid remaining in the detection groove 106 to be easily removed by cleaning. In the example shown in Figs 17A and 17B, the detection groove 106 is disposed parallel to the straight line connecting the analyte liquid supply port leading to the accommodation unit 104 to the discharge port through which the analyte liquid is discharged from the accommodation unit 104.

In the target substance detection plate 101 configured as described above, a plurality of detection structures each constituted by the flow path 103 and the accommodation unit 104 is formed. Thus, the target substance detection chips disposed in the respective accommodation units allow the target substance to be efficiently detected. Furthermore, the detection structures can be allowed to detect different target substances, and a plurality of target substances can be detected during a single operation. Hence, efficient detection tests can be carried out. Moreover, the detection groove 106 in the target substance detection chip 108 is configured to serve as each of the flow path for the analyte liquid and the detection surface for the target substance in the accommodation unit 104. This eliminates the need to separately manufacture the flow path and the detection surface, enabling a reduction in production costs.

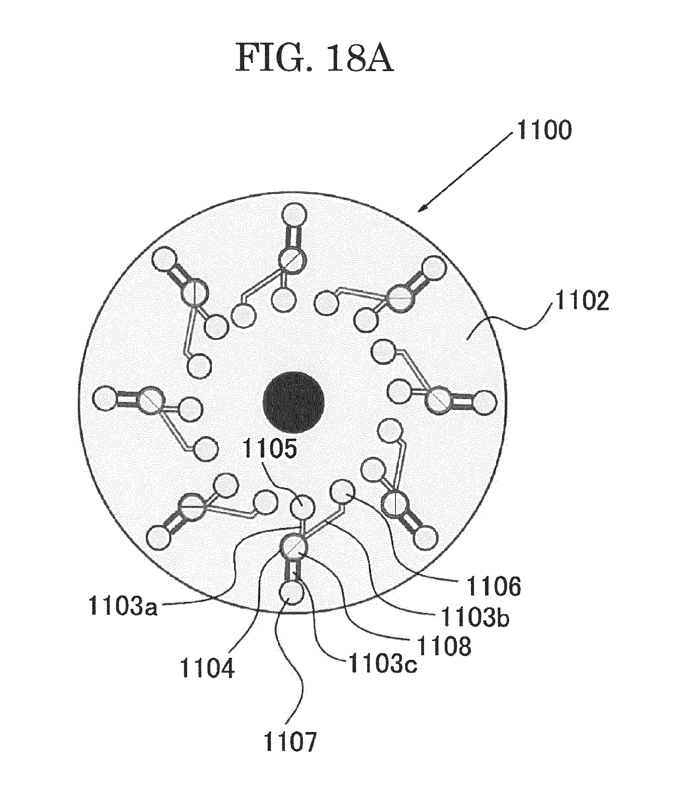

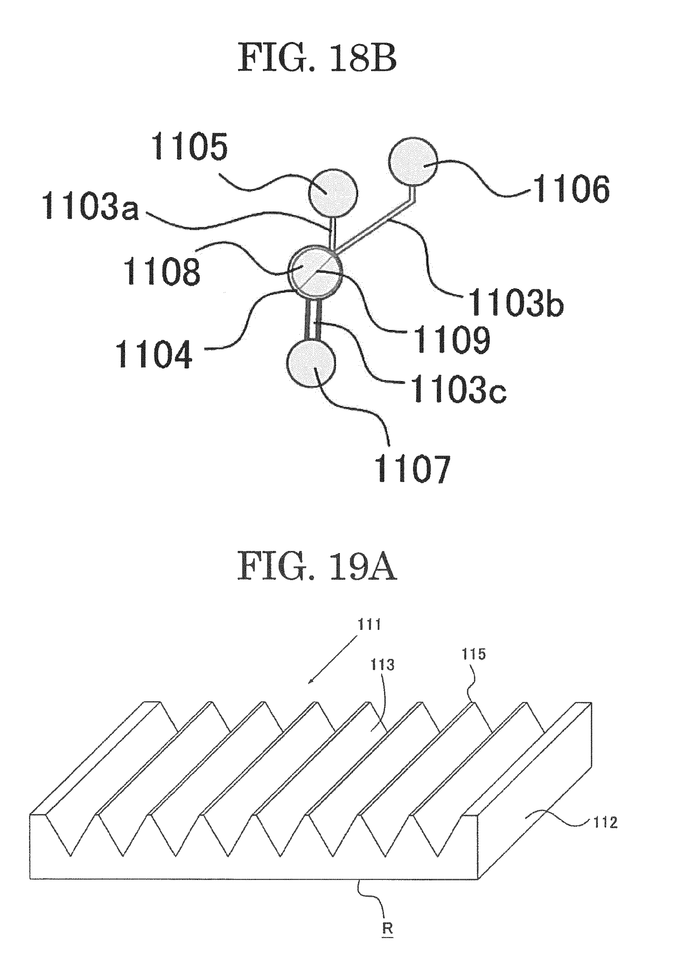

Another embodiment of the target substance detection plate will be described with reference to Figs. 18A and 18B.

As shown in Fig. 18A, a target substance detection plate 1100 consists of a disc-like plate main body 1102. The plate main body 1102 has an accommodation unit 1104 that accommodates a target substance detection chip 1108, an analyte liquid storage unit 1105 that stores an analyte liquid, a cleaning fluid storage unit 1106 that stores a cleaning fluid for removing the analyte liquid by cleaning, a waste liquid storage unit 1107 that stores a waste liquid consisting of the analyte liquid and the cleaning fluid, a flow path 1103a that connects the accommodation unit 1104 to the analyte liquid storage unit 1105, a flow path 1103b that connects the accommodation unit 1104 to the cleaning fluid storage unit 1106, and a flow path 1103c that connects the accommodation unit 1104 to the waste liquid storage unit 1107. A center-of-circle portion of the plate main body 1102 is cut out so that the resulting plate main body 1102 is shaped like a commercially available CD.

The analyte liquid storage unit 1105 and the cleaning fluid storage unit 1106 are disposed closer to the center of the circle of the plate main body 1102 than the accommodation unit 1104. The waste liquid storage unit 1107 is disposed farther from the center of the circle of the plate main body 1102 than the accommodation unit 1104.

Fig. 18B shows an enlarged view showing one detection unit constituted by the accommodation unit 1104, the analyte liquid storage unit 1105, the cleaning fluid storage unit 1106, the waste liquid storage unit 1107, and the flow paths 1103a to 1103c.

A target substance detection chip 1108 accommodated in the accommodation unit 1104 has one detection groove 1109. The flow paths 1103a and 1103b are formed to have a general Y shape with respect to the detection groove 1109. In this case, the detection groove 1109 and a straight line connecting a junction between the accommodation unit 1104 and the flow paths 1103a and 1103b and a junction between the accommodation unit 1104 and the flow path 1103c are arranged such that the arrangement deviates from a parallel state by ±22.5°. The other components are appropriately configured according to the configuration of the target substance detection plate 101.

According to the target substance detection plate 1100, rotationally moving the plate main body 1102 causes a centrifugal force to be generated. This allows the analyte liquid stored in the analyte liquid storage unit 1105 to be delivered to the accommodation unit 1104, allows the cleaning fluid stored in the cleaning fluid storage unit 1106 to be delivered to the accommodation unit 1104, and allows the analyte liquid and cleaning fluid delivered to the accommodation unit 1104 to be delivered to the waste liquid storage unit 1107. Furthermore, the analyte liquid and the cleaning fluid are easily introduced into the detection groove 1109 in the target substance detection chip 1108, allowing detection tests and cleaning to be efficiently carried out.

In the illustrated example, one detection groove 1109 is formed on the target substance detection chip 1108. However, a plurality of detection grooves 1109 may be formed on one target substance detection chip 1108. Additionally, when a plurality of detection units is formed on the plate main body 1102, the detection units each constituted by the accommodation unit 1104, the analyte liquid storage unit 1105, the cleaning fluid storage unit 1106, the waste liquid storage unit 1107, the target substance detection chip 1108, and the flow paths 1103a to 1103c as shown in Fig. 18A, a plurality of detection tests can preferably be carried out at the same time.

Now, an example of an embodiment of the target substance detection chip will be described below with reference to the drawings.

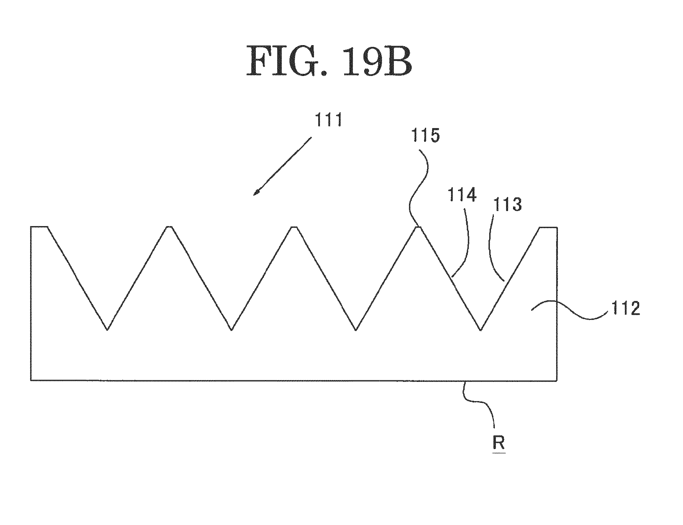

A target substance detection chip 111 according to an embodiment of the present invention shown in Fig. 19A has a configuration in which a detection groove 113 consisting of a groove with a V-shaped cross section is formed in a surface of a transparent base portion 112. Furthermore, the detection groove 113 is formed such that an electric field enhancement layer 114 is disposed on an inner surface of a groove portion formed to have a V-shaped cross section, as shown in Fig. 19B illustrating a cross section of the detection groove 113. An analyte liquid that verifies the presence of a target substance is introduced into the detection groove 113. The uppermost surface, in this case, a surface of the electric field enhancement layer 114, is used as a detection surface for the target substance.

In the electric field enhancement layer 114, light irradiated from a light irradiation unit (not shown in the drawings) excites the surface plasmon resonance or the optical waveguide mode to form a strong electric field in the electric field enhancement layer 114 or near the surface of the electric field enhancement layer 114. The light irradiation unit irradiates the transparent base portion 112 with the light from the side of a surface R of the transparent base portion 112 opposite to the surface of the transparent base portion 112 in which the detection groove 113 is formed.

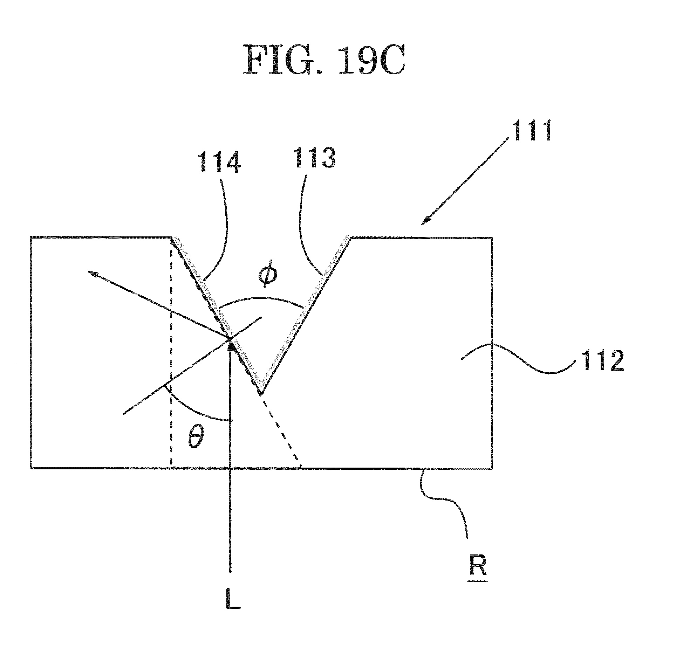

In this case, as shown in Fig. 19C illustrating Fig. 19B, the transparent base portion 112 has an area shown by a dotted line and functioning as a triangular prism to allow light L irradiated from the surface R side to enter the electric field enhancement layer 114 at a particular incident angle. That is, the transparent base portion 112 functions as an optical prism in an SPR sensor and an optical waveguide mode sensor to enhance the electric field in the vicinity of the surface of the electric field enhancement layer 114. This enables a target substance to be detected by this phenomenon. The surface R functions as an incident surface of the prism and thus preferably has high flatness.

The base angle φ of the groove portion of the detection groove 113 shown in Fig. 19C is determined by the incident angle θ of the light L to inclined surfaces forming the groove portion. For example, in the example of the target substance detection chip 111 shown in Fig. 19C, the surface of the transparent base portion 112 in which the detection groove 113 is formed is parallel to the opposite surface R. Two inclined surfaces forming the groove portion are laterally symmetric. The light L from the light irradiation unit perpendicularly enters the surface R. In this case, the base angle φ (°) is selected such that φ = (90° - θ) x 2.

The incident angle θ is determined by excitation conditions for the surface plasmon resonance and the optical waveguide mode. Thus, the base angle φ depends on the refractive index of the formation material for the transparent base portion 112 and the configuration of the electric field enhancement layer 114.

In this case, an excessively low refractive index of the transparent base portion 112 results in the need to increase the incident angle θ and thus the need to reduce the base angle φ. When the flow path is a micro flow path with an opening width of the detection groove 113 of several hundred µm or less, a base angle φ of 30° or less makes formation of the detection groove 113 difficult. Hence, the refractive index of the transparent base portion 112 is preferably 1.38 or greater and more preferably 1.42 or greater. On the other hand, when the size of the detection groove does not particularly affect the degree of difficulty with which the detection groove is machined, the refractive index of the transparent base portion 112 may be lower but is preferably at least higher than the refractive index of water, 1.33.

On the other hand, an excessively high refractive index of the transparent base portion 112 disadvantageously causes the light L to be significantly reflected by the surface R when the light L enters the surface R. Furthermore, candidates for the material of the transparent base portion 112 are limited which have both a high refractive index and high transparency. Hence, the refractive index of the transparent base portion 112 is preferably 4 or less and more preferably 3 or less.

In this example, the detection grooves 113 are formed in parallel in the target substance detection chip 111 as shown in Figs. 19A and 19B. This formation provides a larger surface area of the detection surface than a single detection groove, enabling detection sensitivity for the target substance to be improved.

Furthermore, a spacing 115 may be present between the groove portions of the adjacent detection grooves as described above. The groove portions formed to have the spacing 115 eliminate the need to form groove portions of a stamper forming the groove shape of the transparent base portion 112, i.e., portions of the stamper that make the spacings 115, to subtend an acute angle when the transparent base portion 112 is injection molded. This enables a reduction in production costs.

Additionally, as described above, the spacing 115 is preferably provided with a light blocking portion that attenuates light.

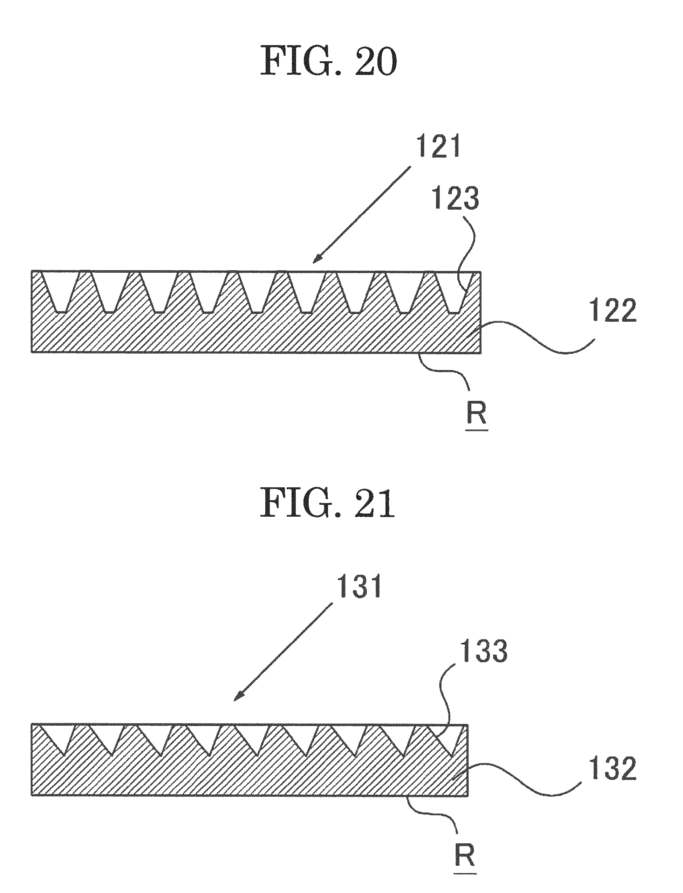

Fig. 20 shows another embodiment of the target substance detection chip. A target substance detection chip 121 has a transparent base portion 122 and a plurality of detection grooves 123. Each of the detection grooves 123 appears in cross section to be shaped like a trapezoid. In such a target substance detection chip, the groove has a bottom portion formed to be flat instead of subtending an acute angle compared to a groove with a V-shaped cross section. Thus, preferably, when the target substance detection chip is cleaned after detection tests are finished, a cleaning fluid flows easily to the bottom portion of the groove, enabling efficient cleaning. However, such a planar portion is preferably provided with a light blocking portion that attenuates light, so as to maximally prevent light from a light irradiation unit from reaching a photodetector side, as is the case with the spacing 115.

The two inclined surfaces in the detection groove constituting the detection groove need not necessarily be laterally symmetric.

For example, as shown in Fig. 21, a detection groove 133 may be formed such that the two inclined surfaces have different gradients. In this case, different luminescence properties are obtained from the respective inclined surfaces. Thus, two different detections can be carried out at the same time.

For example, the right and left surfaces are set to induce electric field enhancement on the surfaces at different wavelengths. For example, the left surface is set such that the surface plasmon thereon is excited by 550-nm light. The right surface is set such that the surface plasmon thereon is excited by 660-nm light. The left surface is set to allow measurement of such an analyte as specifically adsorbs a fluorochrome that emits light when irradiated with 550-nm excitation light. The right surface is set allow measurement of such an analyte as specifically adsorbs a fluorochrome that emits light when irradiated with 660-nm excitation light. Light sources are adapted to emit a 550-nm excitation light beam and a 660-nm excitation light beam, respectively. The light sources alternately irradiate the lights or the lights from the light sources are alternately blocked by filters or the like, to allow signals resulting from the excitation lights to be detected from the respective surfaces. Thus, two analytes can be detected at the same time. In Fig. 21, reference numeral 131 denotes a target substance detection chip, and reference numeral 132 denotes a transparent base portion.

Furthermore, the two inclined surfaces may be formed to have multiple gradients.

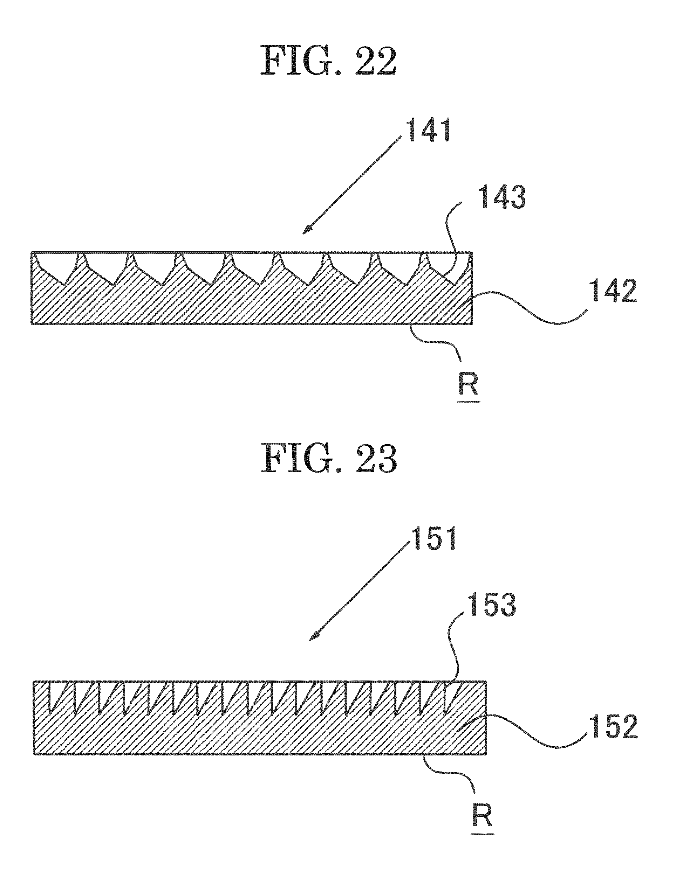

For example, as shown in Fig. 22, a detection groove 143 may be formed such that the two inclined surfaces have multiple gradients. In this case, detections for the respective gradients at the corresponding excitation wavelengths can be carried out at the same time. In Fig. 22, reference numeral 141 denotes a target substance detection chip, and reference numeral 142 denotes a transparent base portion.

Additionally, when fluorescence is detected only by one of the inclined surfaces, the angle subtended by the surface not used for detection does not particularly affect the detection. Thus, the surface not used for detection may have any shape, and as shown in, e.g., Fig. 23, a surface of a detection groove 153 not used for detection may be perpendicularly formed. In Fig. 23 reference numeral 151 denotes a target substance detection chip, and reference numeral 152 denotes a transparent base portion.

### <Light Irradiation Unit>

The light irradiation unit is a unit that irradiates the electric field enhancement layer with light from the side of the surface of the target substance detection chip opposite to the surface of the target substance detection chip in which the detection groove is formed.

The light irradiation unit according to the second embodiment is configured substantially equivalently to the light irradiation unit described in the first embodiment.

That is, the configuration of the light irradiation unit is not particularly limited and can be appropriately selected according to the purpose. The light irradiation unit may be configured by appropriately selecting any of well-known optical members, e.g., light sources such as a laser, a white lamp, and an LED, a collimator that collimates light from the light source, a lens that condenses the light from the light source, and a polarizing plate that polarizes the light from the light source.

In particular, the light irradiation unit is preferably configured to have a polarizing plate that polarizes light emitted from the light source into linearly polarized light.

### <Light Detection Unit>

The light detection unit is configured as a unit that detects fluorescence emitted from the target substance in the analyte liquid present in the detection groove or the fluorescent substance labeling the target substance, as a result of light irradiated from the light irradiation unit.

The light detection unit according to the second embodiment is configured substantially equivalently to the light detection unit described in the first embodiment.

That is, the configuration of the light detection unit is not particularly limited and can be appropriately selected according to the purpose. The light detection unit may be configured by appropriately selecting any of well-known optical members such as photodetectors such as a CCD, a photodiode, and a photomultiplier which detect the fluorescence, an optical fiber that directs the fluorescence to the photodetector, and a condensing lens that condenses and directs the fluorescence to the photodetector.

To determine whether the detected light is derived from the fluorescence emitted from the target substance or the fluorescent substance or from any other light, the photodetector may carry out detection via a wavelength filter that allows only light in the fluorescent wavelength band to pass through.

### <Other members>

The other members are not particularly limited and can be appropriately selected according to the purpose. The other members may include, e.g., a liquid delivery pump. The liquid delivery pump may be a pump that delivers the analyte liquid to the flow path.

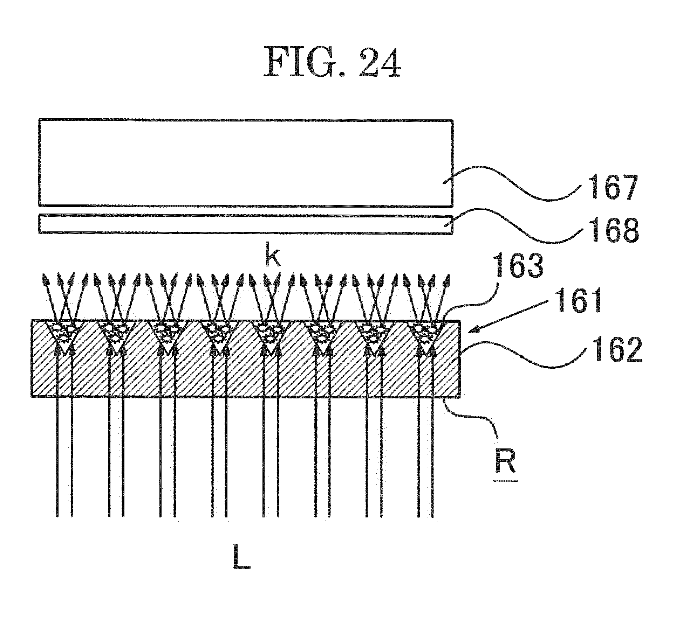

Fig. 24 shows an example of configuration of the target substance detection device for detecting fluorescence emitted from the target substance or the fluorescent substance. In this case, the target substance detection device has a target substance detection chip 161, a light irradiation unit (not shown in the drawings) that irradiates the target substance detection chip 161 with light L from a surface R side thereof, and a photodetector 167 that detects fluorescence k emitted from the target substance or the fluorescent substance, via a wavelength filter 168 that allows only light in the wavelength band of the fluorescence k to pass through. Furthermore, the target substance detection chip 161 has a transparent base portion 162 and a detection groove 163 formed in a surface of the transparent base portion 162.

The irradiated light L may be laser light corresponding to wavelengths in an excitation band for the target substance or the fluorochrome or light made monochromatic by an optical filter or a spectroscope.

In this case, the light irradiation unit is circumferentially rotated in a semi-circle on the surface R side of the target substance detection chip 161 or the target substance detection chip 161 is circumferentially rotated around the fixed light irradiation unit to change the incident angle of the light L to irradiate the electric field enhancement layer in the detection groove 163 with the light L. Then, a phenomenon can be observed in which the luminous intensity increases at a particular angle at which the surface plasmon resonance or the optical waveguide mode is excited. This allows determination of whether the observed luminescence has resulted from the surface plasmon resonance or the optical waveguide mode or the fluorescent substance, upon being irradiated with a stray portion of the light L not involved in the excitation of the surface plasmon resonance or the optical waveguide mode, has emitted light independently of detection of the target substance.

However, a change mechanism for changing the incident angle and a rotation mechanism for circumferentially rotating the target substance detection chip 161 need a movable portion. This may disadvantageously increase the size of the detection device itself. To allow a small, inexpensive device to be configured, a technique is preferably used in which the intensity of fluorescence is observed with the incident angle fixed to a given value to detect the target substance.

When the target substance itself emits the fluorescence k, the presence or absence and the amount of the target substance can be observed by capturing the target substance on the detection surface of the detection groove 163 and observing the presence or absence of luminescence from the target substance and the intensity of the luminescence.

However, many substances fail to exhibit a significant luminescence property. Thus, the target substance is captured on the detection surface of the detection groove 163 and the fluorescent substance is attached to the target substance, and then the luminescence from the fluorescent substance is observed.

A method for attaching the fluorescent substance is not particularly limited, but a well-known technique is applicable. An exemplary method involves binding the fluorescent substance to an antibody specifically adsorbed by the target substance and allowing the antibody with the fluorescent substance to be adsorbed by the target substance.

### (Target Substance Detection Method)

Another target substance detection method according to the present invention is a method for detecting the target substance using the target substance detection device according to the second embodiment of the present invention. The method includes an analyte liquid introduction step, a light irradiation step, and a light detection step.

The analyte liquid introduction step is a step of delivering the analyte liquid through the flow path in the target substance detection plate to introduce the analyte liquid into the detection groove in the target substance detection chip.

The light irradiation step is a step of irradiating the electric field enhancement layer with light from the side of the surface of the target substance detection chip opposite to the surface of the target substance detection chip in which the detection groove is formed.

The light detection step is a step of detecting fluorescence emitted from the target substance in the analyte liquid present in the detection groove or the fluorescent substance labeling the target substance, based on the irradiation with the light carried out in the light irradiation step.

These steps can be appropriately carried out based on the matters described for the target substance detection device.

### Examples

### (Example 1)

First, an example based on the first embodiment of the present invention will be described.

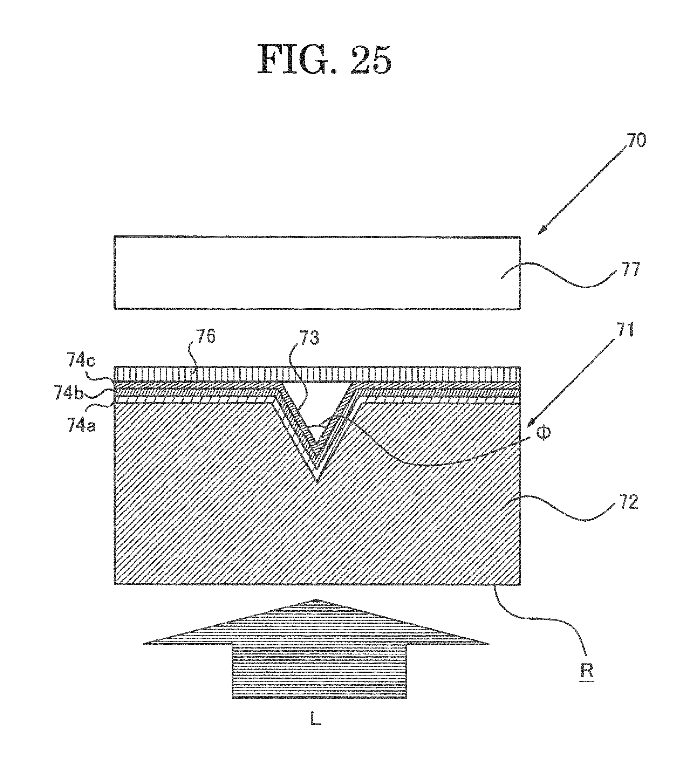

In the example of the present invention, a target substance detection device 70 shown in Fig. 25 was manufactured.

The target substance detection device 70 has a target substance detection chip 71, a light irradiation unit (not shown in the drawings) that irradiates the target substance detection chip 71 with light L from the side of a surface R thereof, and a photodetector 77 that detects fluorescence emitted from the target substance or the fluorescent substance.

The target substance detection chip 71 was manufactured as follows.

First, a plate-like transparent base portion 72 with a groove portion with a V-shaped cross section formed therein was produced by injection molding using polystyrene as a formation material. Two inclined surfaces constituting the groove portion were laterally symmetric, and had a base angle φ of 49°. Furthermore, the groove portion had an opening width of 300 µm. The groove portion was 35 mm in length in the direction in which the analyte liquid flowed. Through-holes (not shown in the drawings) with a diameter of 1 mm were formed at the opposite ends of the groove portion.

Then, chromium was vapor-deposited on a surface of the transparent base portion 72 in which the groove portion was formed so that a film was formed perpendicularly to a flat area in which the groove portion was not formed and so that the film had a thickness of 0.6 nm in the flat area. Thus, a thin chromium film 74a was formed, as an adhesion layer, all over the surface in which the groove portion was formed.

Then, gold was vapor-deposited to a thickness of 100 nm in the flat area to form a thin gold film 74b on the thin chromium film 74a as a surface plasmon excitation layer.

Then, a thin silica glass film was deposited by a sputtering method to a thickness of 49 nm in the flat area to cover a surface of the thin gold film 74b with a transparent dielectric 74c.

Thus, a flow path 73 was formed in the transparent base portion 72. Furthermore, at this time, the thin chromium film 74a and thin gold film 74b stacked on the upper surface of the transparent base portion 72 except for the opening of the flow path 73 served as a light blocking portion.

Then, the opening of the flow path 73 was sealed using, as a lid 76, a cover film containing polymethyl methacrylate as a main component. Thus, the target substance detection chip 71 was manufactured.

Water was injected through a through-hole and filled into the flow path 73. Then, as shown in Fig. 25, the target substance detection chip 71 was irradiated with light from the light irradiation unit of the target substance detection device 70. The light entered the target substance detection chip 71 from the side of a surface R thereof and perpendicularly to the surface R so as to have a beam diameter of about 1 mm. The light irradiation unit was configured in two forms. In one of the forms, the light irradiation unit was constituted by a white light source and a polarizing plate linearly polarizing light emitted from the white light source into p-polarized light. In the other form, the light irradiation unit was constituted by the white light source and a polarizing plate linearly polarizing light emitted from the white light source into s-polarized light.

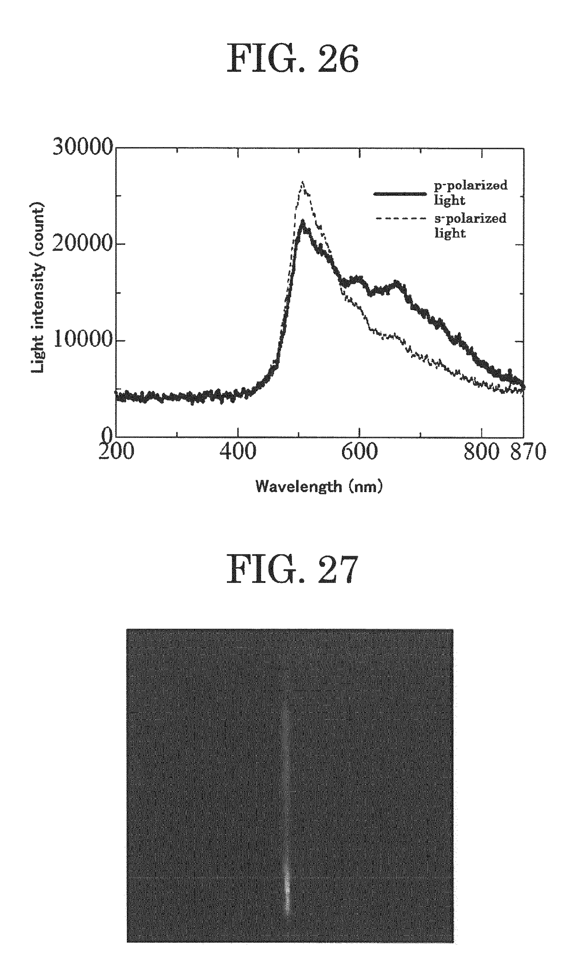

A photodetector 77 disposed opposite the surface of the target substance detection chip 71 with the flow path 73 formed therein was used to measure a transmitted portion of white light irradiated from the surface R side of the target substance detection chip 71 by the light irradiation unit configured in the two forms. Fig. 26 shows the results of the measurement.

Fig. 26 shows the dependency of the intensity of the transmitted light on the wavelength. Fig. 26 indicates that, compared to the s-polarized light, the p-polarized light definitely increases the intensity of the transmitted light in a wavelength region of 570 nm to 870 nm. In view of the fact that the surface plasmon fails to be excited by the s-polarized light, this phenomenon is expected to be caused by significant scatter of the p-polarized incident light resulting from the excitation, by the incident light, of the surface plasmon in the above-described wavelength region.

As described above, the surface plasmon can be easily excited on the target substance detection chip 71 by using the target substance detection device 70 without the need for a complicated step of attaching a prism and a detection chip together as in the case of the conventional art. Furthermore, the excitation of the surface plasmon allows fluorescence from a fluorescent substance to be easily enhanced.

### (Example 2)

As is the case with Example 1, first, a plate-like transparent base portion 72 with a groove portion with a V-shaped cross section formed therein was produced by injection molding using polystyrene as a formation material. The structure of the groove portion is the same as the structure in Example 1. Chromium was vapor-deposited on a surface of the transparent base portion 72 in which the groove portion was formed so that a film was formed perpendicularly to a flat area in which the groove portion was not formed and so that the film had a thickness of 0.6 nm in the flat area. Thus, a thin chromium film 74a was formed as an adhesion layer. Then, gold was vapor-deposited on the chromium layer to a thickness of 120 nm in the flat area to form a thin gold film 74b as a surface plasmon excitation layer. Then, a thin silica glass film (transparent dielectric 74c) was deposited on the gold layer by the sputtering method to a thickness of 49 nm in the flat area. Thus, a flow path 73 was formed in the transparent base portion 72.

Subsequently, the transparent base with the thin films deposited thereon was immersed in a weakly alkaline aqueous solution for 24 hours and then dried. The transparent base was then immersed in an ethanol solution of 0.1 v/v%3-aminopropyltriethoxysilane for 15 hours to modify a surface of the silica glass with reaction active amino group. Subsequently, the transparent base was rinsed in ethanol and then dried, and phosphate buffered saline containing 0.5 mM sulfosuccinimidyl-N-(D-biotinyl)-6-aminohexanate was dropped onto the flow path 73 and left at room temperature for 2 hours. Biotin was introduced onto the surface of the flow path as a substance capturing the target substance. After the above-described process, the opening of the flow path 73 was sealed using, as the lid 76, a cover film containing polymethylmethacrylate as a main component. Thus, the target substance detection chip 71 was manufactured.

A detection target liquid was phosphate buffered silane containing, as a target substance, 100 nM streptavidin with a fluorochrome Alexa 700 (manufactured by Invitrogen Corporation). The detection target liquid was injected and filled into the flow path 73 through a through-hole. Then, the through-hole portion was sealed with a tape, and the transparent base was left at room temperature for 1 hour in order to allow the biotin to capture the streptavidin.

Subsequently, through-hole portion was unsealed, and to remove impurities and the like, the flow path was cleaned five times in phosphate buffered saline containing 0.05 v/v% Triton X-100 (manufactured by NACALAI TESQUE, INC). Then, the flow path 73 was filled with phosphate buffered saline.

The target substance detection chip 71 subjected to the above-described process was irradiated with light L with a diameter of 1 cm using, as a light irradiation unit, an LED with an optical filter which emits light with a wavelength of 680 nm ± 10 nm equipped with a collimator lens and a polarizing plate. Furthermore, a light detection unit was configured by using a cooled CCD camera as the photodetector 77 and installing, in front of the CCD camera, an optical filter that allows light of wavelength 710 nm or greater to pass through and an optical filter that allows light of wavelength 720 nm or greater to pass through. An exposure time was set to 60 seconds.

When p-polarized light was irradiated from the light irradiation unit, fluorescence from Alexa 700 was successfully observed which shone along the flow path and which appeared as a white line in a photograph shown in Fig. 27. On the other hand, when s-polarized light was irradiated from the light irradiation unit, no fluorescence from Alexa 700 was observed. The surface plasmon is excited only by irradiation with p-polarized light, and thus, the observation results indicate that the fluorescence from the fluorochrome was enhanced by excitation of surface plasmon by the surface plasmon excitation layer in the detection surface in the flow path 73, allowing the analyte to be sensitively detected.

### (Example 3)

Now, an example based on the second embodiment relating to the target substance detection plate according to the present invention will be described.

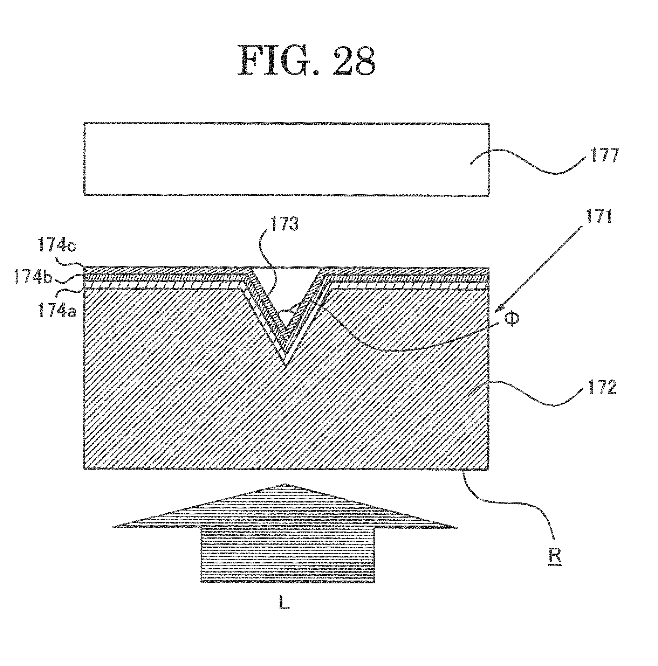

To confirm the effectiveness of the second embodiment of the present invention, a prototype was produced which had a target substance detection chip 171, a light irradiation unit (not shown in the drawings) irradiating the detection chip 171 with light L from the side of a surface R thereof, and a photodetector 177 detecting fluorescence emitted from the target substance or the fluorescent substance (see Fig. 28).

In this case, the target substance detection chip 171 was manufactured as follows.

First, a plate-like transparent base portion 172 with a groove portion with a V-shaped cross section formed therein was produced by injection molding using polystyrene as a formation material. Two inclined surfaces constituting the groove portion were laterally symmetric, and had a base angle φ of 49°. Furthermore, the groove portion had an opening width of 300 µm.

Then, chromium was vapor-deposited on a surface of the transparent base portion 172 in which the groove portion was formed so that a film was formed perpendicularly to a flat area in which the groove portion was not formed and so that the film had a thickness of 0.6 nm in the flat area. Thus, a thin chromium film 174a was formed, as an adhesion layer, all over the surface in which the groove portion was formed.

Then, gold was vapor-deposited to a thickness of 100 nm in the flat area to form a thin gold film 174b on the thin chromium film 174a as a surface plasmon excitation layer.

Then, a thin silica glass film was deposited by the sputtering method to a thickness of 49 nm in the flat area to cover a surface of the thin gold film 174b with a transparent dielectric 174c.

Thus, a detection groove 173 with a groove shape approximately the same as the shape of the groove portion was formed in the transparent base portion 172. Furthermore, at this time, the thin chromium film 174a and thin gold film 174b stacked on the upper surface of the transparent base portion 172 except for the opening of the detection groove 173 served as a light blocking portion.

Thus, the target substance detection chip 171 was manufactured.

The target substance detection chip 171 was filled with water through the detection groove 173. As shown in Fig. 28, the target substance detection chip 171 was irradiated with light from the light irradiation unit. The light entered the target substance detection chip 171 from the side of a surface R thereof and perpendicularly to the surface R so as to have a beam diameter of about 1 mm. The light irradiation unit was configured in two forms. In one of the forms, the light irradiation unit was constituted by a white light source and a polarizing plate linearly polarizing light emitted from the white light source into p-polarized light. In the other form, the light irradiation unit was constituted by the white light source and a polarizing plate linearly polarizing light emitted from the white light source into s-polarized light.

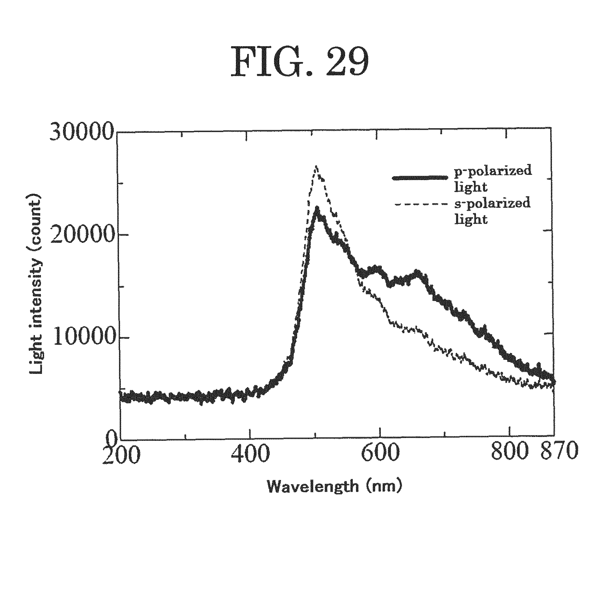

A photodetector 177 disposed opposite the surface of the target substance detection chip 171 with the detection groove 173 formed therein was used to measure a transmitted portion of white light irradiated from the surface R side of the target substance detection chip 171 by the light irradiation unit configured in the two forms. Fig. 29 shows the results of the measurement.

Fig. 29 shows the dependency of the intensity of the transmitted light on the wavelength. Fig. 29 indicates that, compared to the s-polarized light, the p-polarized light definitely increases the intensity of the transmitted light in a wavelength region of 570 nm to 870 nm. In view of the fact that the surface plasmon fails to be excited by the s-polarized light, this phenomenon is expected to be caused by significant scatter of the p-polarized incident light resulting from the excitation, by the incident light, of the surface plasmon in the above-described wavelength region.

As described above, the surface plasmon can be easily excited on the target substance detection chip 171 by using the target substance detection chip 171 without the need for a complicated step of attaching a prism and a detection chip together as in the case of the conventional art. Furthermore, the excitation of the surface plasmon allows fluorescence from a fluorescent substance to be easily enhanced. Additionally, the target substance can be efficiently detected by using the target substance detection plate that accommodates the target substance detection chip 171.

### (Example 4)

As is the case with Example 3, first, a plate-like transparent base portion 172 with a groove portion with a V-shaped cross section formed therein was produced by injection molding using polystyrene as a formation material. The structure of the groove portion is the same as the structure in Example 3. Chromium was vapor-deposited on a surface of the transparent base portion 172 in which the groove portion was formed so that a film was formed perpendicularly to a flat area in which the groove portion was not formed and so that the film had a thickness of 0.6 nm in the flat area. Thus, a thin chromium layer 174a was formed as an adhesion layer. Then, gold was vapor-deposited on the chromium layer to a thickness of 120 nm in the flat area to form a thin gold layer 174b as a surface plasmon excitation layer. Then, a thin silica glass film (transparent dielectric layer 174c) was deposited on the gold layer by the sputtering method to a thickness of 49 nm in the flat area. Thus, a detection groove 173 was formed in the transparent base portion 172.

Subsequently, the transparent base with the thin films deposited thereon was immersed in a weakly alkaline aqueous solution for 24 hours and then dried. The transparent base was then immersed in an ethanol solution of 0.1 v/v%3-aminopropyltriethoxysilane for 15 hours to modify a surface of the silica glass with reaction active amino group. Subsequently, the transparent base was rinsed in ethanol and then dried, and phosphate buffered saline containing 0.5 mM sulfosuccinimidyl-N-(D-biotinyl)-6-aminohexanate was dropped onto the detection groove 173 and left at room temperature for 2 hours. Biotin was introduced onto the surface of the detection groove as a substance capturing the target substance. Thus, the target substance detection chip 171 was manufactured.

Then, a target substance detection plate 1100 shown in Fig. 18A was produced as follows.

A COP (cyclic polyolefin) substrate was utilized as a formation base material for a plate main body 1102. Based on a CAD design, the COP substrate was cut using an NC (Numerical Control) processing machine, with cutting tools of diameter 0.01 mm to 4 mm appropriately changed with one another. Thus, the plate main body 1102 was produced which had an accommodation unit 1104, an analyte liquid storage unit 1105, a cleaning fluid storage unit 1106, a waste liquid storage unit 1107, and flow paths 1103a to 1103c.

The accommodation unit 1104 was shaped like a cylinder with a diameter of 5.2 mm and a depth of 1.6 mm.

The target substance detection chip 171 (the plate thickness of the chip was 1.5 mm) was cut into a cylinder with a diameter of 5.2 mm by machining by the NC processing machine. The resulting target substance detection chip 171 was incorporated into the accommodation unit 1104.

Before the incorporation, a back surface of the target substance detection chip 171 was dulled so that the target substance detection chip 171 was easily incorporated into the accommodation unit 1104.

Subsequently, the entire surface of the plate main body 1102 was sealed (capped) with a pressure-sensitive adhesive transparent sheet so as to cover all the flow paths 1103a to 1103c. Then, the seal was partly removed using a CO₂ laser marker, for the purpose of injection of an analyte liquid or air vent.

Subsequently, when the boundary surface of the incorporated target substance detection chip 171 was observed with a confocal microscope, the gap between the boundary surface and a surface of the plate main body 1102 (i.e., a back surface of the seal) was 50 µm. When the analyte liquid is introduced into the gap portion, a fluorescent label attached to the target substance adsorbed by an inner wall of the detection groove 173 emits intense light due to an electric field enhancing effect, allowing the target substance to be sensitively detected. Furthermore, the gap is preferably narrow and is about 0 µm to 200 µm. This is because the thinned gap portion facilitates an antigen-antibody reaction to enable detection in a short time.

The flow path 1103a from the analyte liquid storage unit 1105 to the accommodation unit 1104 was 500 µm in width and 100 µm in depth. The flow path 1103b from the cleaning fluid storage unit 1106 to the accommodation unit 1104 was 200 µm in width and 50 µm in depth. The flow path 1103c from the accommodation unit 1104 to the waste liquid storage unit 1107 was 30 µm in width and 50 µm in depth.

A detection target liquid was phosphate buffered silane containing, as a target substance, 100 nM streptavidin with a fluorochrome Alexa 700 (manufactured by Invitrogen Corporation). The detection target liquid was injected and filled into the detection groove 173 via the flow path 1103a. Then, the transparent base was left at room temperature for 1 hour in order to allow biotin to capture streptavidin.

Subsequently, for removal of impurities and the like, phosphate buffered saline containing 0.05 v/v% Triton X-100 (manufactured by NACALAI TESQUE, INC) was injected into the detection groove 173 via 1103b, and the detection groove 173 was cleaned. Then, the detection groove 173 was filled with phosphate buffered saline.

The target substance detection plate 1100 subjected to the above-described process was irradiated with light using, as a light irradiation unit, an LED with an optical filter which emits light with a wavelength of 680 nm ± 10 nm equipped with a collimator lens and a polarizing plate. Furthermore, a light detection unit was configured by using a cooled CCD camera as the photodetector 177 and installing, in front of the CCD camera, an optical filter that allows light of wavelength 710 nm or greater to pass through and an optical filter that allows light of wavelength 720 nm or greater to pass through. The exposure time was set to 60 seconds.

When p-polarized light was irradiated from the light irradiation unit, fluorescence from Alexa 700 was successfully observed. On the other hand, when s-polarized light was irradiated from the light irradiation unit, no fluorescence from Alexa 700 was observed. The surface plasmon is excited only by irradiation with p-polarized light, and thus, the observation results indicate that the fluorescence from the fluorochrome was enhanced by excitation of surface plasmon by the surface plasmon excitation layer in the detection surface in the detection groove 173, allowing the analyte to be sensitively detected.

### Reference Signs List

1, 11, 21, 21', 31, 41, 51, 61, 71, 81: Target substance detection chip
2, 12, 22, 22', 32, 42, 52, 62, 72, 82: Transparent base portion
3, 13, 23, 23', 33, 43, 53, 63, 73, 83: Flow path
4: Electric field enhancement layer
15, 15': Through-hole
16, 26, 26', 36, 46, 56, 66, 76, 86: Lid
30, 60, 70: Target substance detection device
37, 37', 67, 77, 206, 309, 405, 509: Photodetector
68: Wavelength filter
69: Light blocking portion
74a: Thin chromium film
74b: Thin gold film
74c: Transparent dielectric
201, 306, 401a, 506a: Transparent substrate
401, 506: Detection plate
401b, 506b: Thin layer
401c, 506c: Optical waveguide layer
302A, 302B, 502A, 502B; Optical fiber
304, 503: Collimator lens
205, 305, 404, 504: Polarizing plate
203, 303, 402, 505: Optical prism
308, 507: Condensing lens
309A, 508: Spectroscope
200, 300: SPR sensor
202, 307: Thin metal layer
204, 301, 403, 501: Light source
210A, 310A, 410A: Incident light
210B, 310B, 410B: Reflected light
400, 500: Optical waveguide mode sensor
R: Surface
L, L1, L2: Light
k: Fluorescence
θ: Incident angle
φ: Base angle
101, 1100: Target substance detection plate
102, 1102: Plate main body
103, 1103a, 1103b, 1103c: Flow path
104, 1104: Accommodation unit
105, 1105: Analyte liquid storage unit
104', 105', 1107: Waste liquid storage unit
106, 113, 123, 133, 143, 153, 163, 173, 1109: Detection groove
107, 112, 122, 132, 142, 152, 162, 172: Transparent base portion
108, 111, 121, 131, 141, 151, 161, 171, 1108: Target substance detection chip
109: Lid
110: Light source
114: Electric field enhancement layer
115: Spacing
167, 177: Photodetector
168: Wavelength filter
174a: Thin chromium film
174b: Thin gold film
174c: Transparent dielectric layer
1106: Cleaning fluid storage unit
A: Direction

## Claims

1. A target substance detection chip, comprising:
a plate-like transparent base portion which allows light to pass therethrough; and
a flow path which is formed in one surface of the transparent base portion as a groove and through which an analyte liquid verifying a presence of a target substance is delivered in a length direction of the groove,
wherein the flow path is formed such that at least an electric field enhancement layer is disposed on an inner surface of a groove portion formed to at least partly have inclined surfaces appearing in cross section to be inclined at a gradient to the surface of the transparent base portion, and
wherein a part or entirety of an uppermost surface of the groove which contacts the analyte liquid serves as a detection surface for the target substance.

2. The target substance detection chip according to claim 1, wherein a surface of the transparent base portion opposite to the surface of the transparent base portion in which the flow path is formed is formed to be flat.

3. The target substance detection chip according to claim 1 or 2, wherein a right groove side surface and a left groove side surface forming the groove portion are formed to be laterally symmetric.

4. The target substance detection chip according to any one of claims 1 to 3, wherein the electric field enhancement layer is formed such that a surface plasmon excitation layer that causes surface plasmon resonance is disposed on the groove portion.

5. The target substance detection chip according to claim 4, wherein a formation material for the surface plasmon excitation layer contains at least one of gold, silver, copper, platinum, and aluminum.

6. The target substance detection chip according to claim 4 or 5, wherein a surface of the surface plasmon excitation layer is covered with a transparent dielectric.

7. The target substance detection chip according to any one of claims 1 to 3, wherein the electric field enhancement layer is formed of: a thin layer formed of a metal material or a semiconductor material; and an optical waveguide layer formed of a transparent material, the thin layer and the optical waveguide layer being disposed on the groove portion in this order.

8. The target substance detection chip according to claim 7, wherein the metal material contains at least one of gold, silver, copper, platinum, and aluminum.

9. The target substance detection chip according to claim 7, wherein the semiconductor material is silicon.

10. The target substance detection chip according to any one of claims 7 to 9, wherein the optical waveguide layer is formed of silica glass.

11. The target substance detection chip according to any one of claims 1 to 10, wherein the detection surface is surface-treated so as to capture the target substance.

12. The target substance detection chip according to any one of claims 1 to 11, wherein a lid is disposed on the surface of the transparent base portion in which the flow path is formed so as to block an opening of the flow path.

13. The target substance detection chip according to claim 12, wherein the lid comprises a seal material or a plate material, which is formed of a transparent resin material or a transparent glass material.

14. The target substance detection chip according to claim 12, wherein the lid includes a reflection material, a seal material containing a reflection layer, or a plate material containing a reflection layer.

15. A target substance detection device, comprising:
the target substance detection chip according to any one of claims 1 to 14;
a light irradiation unit configured to irradiate the electric field enhancement layer with light from a side of a surface of the target substance detection chip opposite to a surface of the target substance detection chip in which a flow path is formed; and
a light detection unit configured to detect light reflected from the electric field enhancement layer.

16. A target substance detection device, comprising:
the target substance detection chip according to any one of claims 1 to 14;
a light irradiation unit configured to irradiate the electric field enhancement layer with light from a side of a surface of the target substance detection chip opposite to a surface of the target substance detection chip in which a flow path is formed; and
a light detection unit configured to detect fluorescence emitted from the target substance or a fluorescent substance labeling the target substance in the analyte liquid present in the flow path, based on the irradiation with the light.

17. The target substance detection device according to claim 15 or 16, wherein the light irradiation unit comprises:
a light source; and
a polarizing plate configured to polarize light emitted from the light source into linearly polarized light.

18. A target substance detection method for detecting a target substance using the target substance detection device according to claim 15, the method comprising:
delivering the analyte liquid verifying a presence of the target substance through the flow path in the target substance detection chip;
irradiating the electric field enhancement layer with light from a side of a surface of the target substance detection chip opposite to a surface of the target substance detection chip in which the flow path is formed; and
detecting light reflected from the electric field enhancement layer.

19. A target substance detection method for detecting a target substance using the target substance detection device according to claim 16, the method comprising:
delivering the analyte liquid verifying a presence of the target substance through the flow path in the target substance detection chip;
irradiating the electric field enhancement layer with light from a side of a surface of the target substance detection chip opposite to a surface of the target substance detection chip in which the flow path is formed; and
detecting fluorescence emitted from the target substance or a fluorescent substance labeling the target substance in the analyte liquid present in the flow path, based on the irradiation with the light.

20. A target substance detection plate, comprising:
a translucent plate main body in which one or more accommodation units and flow paths are formed, the accommodation unit having a shape of a recess each accommodating the target substance detection chip according to any one of claims 1 to 11 which detects a target substance, the flow path allowing the analyte liquid verifying a presence of the target substance to be delivered to the accommodation unit; and
the target substance detection chip accommodated in the accommodation unit,
wherein the flow path in the target substance detection chip is connected to the flow path in the plate main body to form a detection groove into which the analyte liquid is introduced.

21. The target substance detection plate according to claim 20, wherein the plate main body comprises a disc-like member.

22. The target substance detection plate according to claim 20 or 21, wherein the plate main body is formed of a disc-like member and comprises:
an analyte liquid storage unit configured to store the analyte liquid and a cleaning fluid storage unit configured to store a cleaning fluid, the analyte liquid storage unit and the cleaning fluid storage unit being disposed at positions closer to a center of a circle of the disc-like member than the accommodation unit; and
a waste liquid storage unit disposed at a position farther from the center of the circle than the accommodation unit and configured to store a waste liquid including the analyte liquid and the cleaning fluid, and
each of the analyte liquid storage unit, the cleaning fluid storage unit, and the waste liquid storage unit is connected to the accommodation unit via the flow path in the plate main body through which the analyte liquid, the cleaning fluid, and the waste liquid are delivered.

23. The target substance detection plate according to any one of claims 20 to 22, wherein the detection groove appears in cross section to be shaped like a trapezoid.

24. The target substance detection plate according to claim 23, wherein a light blocking portion is formed on a bottom surface of the detection groove.

25. The target substance detection plate according to any one of claims 20 to 24, wherein a plurality of detection grooves is formed in parallel with respect to one target substance detection chip.

26. The target substance detection plate according to claim 25, wherein a spacing is provided between groove portions of the adjacent detection grooves.

27. The target substance detection plate according to claim 26, wherein the light blocking portion is formed in an area forming the spacing between the groove portions.

28. A target substance detection device, comprising:
the target substance detection plate according to any one of claims 20 to 27;
a light irradiation unit configured to irradiate the electric field enhancement layer with light from a side of a surface of the target substance detection chip opposite to a surface of the target substance detection chip in which the detection groove is formed; and
a light detection unit configured to detect fluorescence emitted from the target substance or a fluorescent substance labeling the target substance in the analyte liquid present in the detection groove, based on the irradiation with the light.

29. A target substance detection method for detecting a target substance using the target substance detection device according to claim 28, the method comprising:
delivering the analyte liquid through the flow path in the plate main body of the target substance detection plate to introduce the analyte liquid into the detection groove in the target substance detection chip;
irradiating the electric field enhancement layer with light from a side of a surface of the target substance detection chip opposite to a surface of the target substance detection chip in which the detection groove is formed; and
detecting fluorescence emitted from the target substance or a fluorescent substance labeling the target substance in the analyte liquid present in the detection groove, based on the irradiation with the light.
